# EUROPEAN PATENT SPECIFICATION

(11) **EP 4 373 918 B1**
(45) Date of publication and mention of the grant of the patent: **09.09.2026**
(21) Application number: 22747460.8
(22) Date of filing: 22.07.2022
(51) Int. Cl.: C12N 5/077, A23L 13/00, C12N 5/00

(54) **SERUM-FREE MEDIA FOR PRODUCING ADIPOCYTES FOR ANIMAL CONSUMPTION**
SERUMFREIE MEDIEN ZUR HERSTELLUNG VON ADIPOZYTEN FÜR DEN TIERISCHEN VERZEHR
MILIEUX SANS SÉRUM POUR LA PRODUCTION D'ADIPOCYTES POUR LA CONSOMMATION ANIMALE

(30) Priority: 22.07.2021 NL 2028813
(43) Date of publication of application: 29.05.2024
(73) Proprietor: Mosa Meat B.V., 6229 PM Maastricht (NL)
(72) Inventor: MITIC, Rada, 6229 PM Maastricht (NL); CANTONI, Federica, 6229 PM Maastricht (NL); HUBALEK, Sophie Claire, 6229 PM Maastricht (NL); JACKISCH, Laura, 6229 PM Maastricht (NL); MEI, Arianna, 6229 PM Maastricht (NL); MELKE, Johanna, 6229 PM Maastricht (NL)
(74) Representative: Moura, Miguel
(86) International application number: PCT/NL2022/050431
(87) International publication number: WO 2023/003470

(56) References cited:
- WO-A1-2018/227016
- DEVIREDDY LAXMINARAYANA R ET AL: "A serum-free medium formulation efficiently supports isolation and propagation of canine adipose-derived mesenchymal stem/stromal cells", PLOS ONE, 1 January 2019 (2019-01-01), United States, pages e0210250 - e0210250, XP055905134, Retrieved from the Internet <URL:https://journals.plos.org/plosone/article/file?id=10.1371/journal.pone.0210250&type=printable> [retrieved on 20220324], DOI: 10.1371/journal.pone.0210250
- GHONIEM AMIR-ALEXANDER ET AL: "Improved adipogenic in vitro differentiation: comparison of different adipogenic cell culture media on human fat and bone stroma cells for fat tissue engineering", ANATOMY & CELL BIOLOGY, 1 June 2015 (2015-06-01), Korea (South), pages 85 - 94, XP055905136, Retrieved from the Internet <URL:https://www.ncbi.nlm.nih.gov/pmc/articles/PMC4488646/pdf/acb-48-85.pdf> [retrieved on 20220324], DOI: 10.5115/acb.2015.48.2.85
- WANG WENSHAN ET AL: "A PRDM16-Driven Metabolic Signal from Adipocytes Regulates Precursor Cell Fate", CELL METABOLISM, vol. 30, no. 1, 2 July 2019 (2019-07-02), pages 174, XP085726427, ISSN: 1550-4131, DOI: 10.1016/J.CMET.2019.05.005
- MARIANA P HANGA ET AL: "Bioprocess development for scalable production of cultivated meat", BIOTECHNOLOGY AND BIOENGINEERING, JOHN WILEY, HOBOKEN, USA, vol. 117, no. 10, 23 July 2020 (2020-07-23), pages 3029 - 3039, XP071052536, ISSN: 0006-3592, DOI: 10.1002/BIT.27469
- O'NEILL EDWARD N. ET AL: "Considerations for the development of cost?effective cell culture media for cultivated meat production", vol. 20, no. 1, 5 December 2020 (2020-12-05), US, pages 686 - 709, XP055779405, ISSN: 1541-4337, Retrieved from the Internet <URL:https://onlinelibrary.wiley.com/doi/full-xml/10.1111/1541-4337.12678> DOI: 10.1111/1541-4337.12678

## Description

### FIELD OF THE INVENTION

The invention is in the field of serum-free, chemically defined media that allow for the differentiation of adipogenic progenitor (precursor) cells into adipocytes for animal, preferably human, consumption. The serum-free media of the invention allow for the differentiation of mammalian adipogenic progenitor cells, such as bovine, ovine or porcine adipogenic progenitor cells, into adipocytes and do not require the use of conventional food-incompatible differentiation inducers. The cultured adipocytes obtained by the methods of the invention can be used in the manufacturing of cultured fat products and cultured meat products for human consumption.

### BACKGROUND OF THE INVENTION

For the culturing and differentiation of animal cells, serum is traditionally used as part of a medium. Serum provides important nutrients, vitamins, growth factors, proteins and electrolytes. An especially widely used serum is fetal bovine serum (FBS), which is rich in growth factors. Although serum is beneficial in the proliferation and differentiation of animal cells, there are some clear disadvantages to the use of serum.

The use of serum in products for human consumption may introduce a health risk, as serum may contain bacteria, viruses and prions. In addition, the composition of serum depends on the source from which it is obtained, different batches of serum may lead to different or non-reproducible results in relation to cell culture. An additional ethical consideration is the well-being of animals used for obtaining serum. Also, the use of serum is expensive and introduces a cost burden when used on a large scale. Therefore, demand has arisen for alternatives to serum.

However, it remains a challenge to provide for serum-free and chemically defined media that at least achieve similar results in terms of adipogenic differentiation potential as compared to conventional media. Serum-free media for the production of cell culture-based fat or meat products do not only need to allow for sufficient differentiation levels compared to traditional serum-based media, but should also not include components that are not safe for human consumption and therefore incompatible with human food regulations. Culture media for differentiating adipogenic progenitor cells often include as differentiation inducers the compounds 3-isobutyl-1-methylxanthine (IBMX), dexamethasone and rosiglitazone, which are a hazard to human health, and therefore not suitable and acceptable in human food manufacturing processes.

There is a need in the art for serum-free adipogenic differentiation media that provide for advantageous differentiation of adipogenic precursor cells and which can be applied in a cell-culture based human food manufacturing method.

Some prior art documents to note:
- Devireddy Laxminarayana R. et al: "A serum-free medium formulation efficiently supports isolation and propagation of canine adipose-derived mesenchymal stem/stromal cells", PloS one, 2019-01-01, pages e0210250-e0210250: describes serum-free media for adipogenic differentiation comprising insulin,hydrocortisone, progesterone, bFGF, EGF and putrescine.Ghoniem Amir-Alexander et al: "Improved adipogenic in vitro differentiation: comparison of different adipogenic cell culture media on human fat and bone stroma cells for fat tissue engineering", Anatomy & cell biology, 2015-06-01), pages 85-94: compares adipogenic media comprising, among other things, indomethacin/insulin or hydrocortison/insulin.
- Wang Wenshan et al: "A PRDM16-Driven Metabolic Signal from Adipocytes Regulates Precursor Cell Fate", CELL METABOLISM, issue 30, nr. 1, 2019-07-02, page 174: describes adipocyte differentiation medium using a cocktail comprising PPARgamma inhibitors (DMEM/F 12 containing 10% FBS, isobutylmethylxanthine, indomethacin, dexamethasone, insulin, T3, and rosiglitazone).

- WO 2018/227016 A1 (Wild Type Inc) 2018-12-13: describes ex vivo meat production comprising adipocytes and muscle cells. Various suitable media are disclosed in a generic fashion.
- Mariana P Hanga et al: "Bioprocess development for scalable production of cultivated meat", Biotechnology and Bioengineering, issue 117, nr. 10, 2020-07-23, pages 3029-3039: discloses that bovine adipose derived stem cells (bASCs) were used as starting cells for artificial meat production due to their ability to differentiate towards both fat and muscle.
- O'Neill Edward N. et al: "Considerations for the development of cost-effective cell culture media for cultivated meat production", Comprehensive Reviews in Food Science and Food Safety, issue 20, nr. 1, 2020-12-05, pages 686-709: describes the process for the development of cost-effective serum-free media for artificial meat production.

### SUMMARY OF THE INVENTION

The present inventors discovered amongst others a serum-free, chemically defined adipogenic differentiation medium that can advantageously be used in the culture and differentiation of mammalian adipogenic progenitor cells and which can be applied in a cell-culture based human food manufacturing methods. It was further discovered that such a serum-free medium outperforms traditional and conventional serum-based differentiation media that employ adipogenic differentiation inducers such as the often used 3-isobutyl-1-methylxanthine (IBMX), dexamethasone and rosiglitazone, in terms of differentiation potential. It was also discovered that adipogenic progenitor cells of different mammalian species can be successfully differentiated.

Therefore, the invention provides in a first aspect a method for differentiating an adipogenic progenitor cell, comprising the step of: - culturing an adipogenic progenitor cell in a a chemically defined serum-free medium for differentiating an adipogenic progenitor cell, wherein the serum-free medium comprises: a basal medium; - at least one peroxisome proliferator-activated receptor gamma (PPARy) agonist said at least one PPARy agonist selected from the group consisting of indomethacin, amorfrutin B, magnolol and honokiol, preferably indomethacin or magnolol; - hormones consisting of insulin and hydrocortisone; - cytokines and/or growth factors consisting of bone morphogenetic protein 4 (BMP4) and epidermal growth factor (EGF); and - ascorbic acid or a derivative thereof.

In a preferred embodiment of said method for differentiating, said serum-free medium comprises bone morphogenetic protein 4 (BMP4) and epidermal growth factor (EGF), cytokines and/or growth factors; and wherein said serum-free medium optionally further comprises fibroblast growth factor (FGF).

In another preferred embodiment of said method for differentiating, said serum-free medium further comprises at least one biogenic amine such as putrescine.

In another preferred embodiment of said method for differentiating, said serum-free medium further comprises a source of lipids such as a source of saturated and/or unsaturated fatty acids, preferably supplemented to said basal medium in the form of a concentrate.

In another preferred embodiment of said method for differentiating, said basal medium is DMEM, Ham's F-12 or a mixture thereof.

In another preferred embodiment of said method for differentiating, said serum-free medium does not comprise a differentiation inducer selected from the group consisting of isobutyl-methyl-xantane (IBMX), dexamethasone and/or a thiazolidinedione such as rosiglitazone, pioglitazone, lobeglitazone, cigilitazone, darglitazone, englitazone, netoglitazone, rivoglitazone, troglitazone and/or balaglitazone.

In another preferred embodiment of said method for differentiating, said serum-free medium comprises a source of energy that allows for differentiation of said adipogenic progenitor cell, said source of energy is an (energy-containing) substrate involved in at least one energy metabolism pathway such as glycolysis, mitochondrial respiration and/or pentose phosphate pathway and/or said source of energy is at least one sugar, preferably a monosaccharide or a disaccharide, such as glucose or galactose, optionally in combination with a source of glutamine, a pyruvate, acetateand/or alpha-ketoglutarate (aKG).

In a futher preferred embodiment of said method for differentiating, said serum-free medium for differentiating further comprises:
- hydrocortisone; - insulin; - bone morphogenetic protein 4 (BMP4); - ascorbic acid or a derivative thereof; and - a basal medium, preferably DMEM/F 12; and wherein said serum-free medium for differentiating optionally further comprises - putrescine, - a source of lipids, preferably wherein said source of lipids is a source of saturated and unsaturated fatty acids, - progesterone; and/or - HEPES.

In another preferred embodiment of said method for differentiating, said method for differentiating an adipogenic progenitor cell is a method for producing a cultured adipocyte for animal, preferably human, consumption by differentiating said adipogenic progenitor cell into an adipocyte.

In another preferred embodiment of said method for differentiating, said method is a method for proliferating an adipogenic progenitor cell followed by differentiating proliferated adipogenic progenitor cells, wherein said method further comprises, prior to differentiating said adipogenic progenitor cell, a step of: - culturing an adipogenic progenitor cell in a serum-free medium for proliferating adipogenic progenitor cells, to thereby provide proliferated adipogenic progenitor cells.

In another preferred embodiment of said method for differentiating, said adipogenic progenitor cell is a mammalian adipogenic progenitor cell, preferably a bovine adipogenic progenitor cell such as a muscle-derived bovine adipogenic progenitor cell such as a fibro-adipogenic progenitor (FAP) cell.

In another preferred embodiment of said method for differentiating, said culturing of said adipogenic progenitor cell in said serum-free medium for differentiating is performed under conditions that allow for differentiation of said adipogenic progenitor cell into an adipocyte.

In another preferred embodiment of said method for differentiating, said method further comprises the step of: - incorporating said cultured adipocyte into a food product for animal, preferably human, consumption.

In another aspect, the invention provides a serum-free medium for differentiating an adipogenic progenitor cell, wherein said medium is as defined in any one of the preceding aspects and/or embodiments relating to a method for differentiating an adipogenic progenitor cell.

In another aspect, the invention provides a cultured fat product for animal, preferably human, consumption, comprising adipocytes obtainable by a method for differentiating according to the invention.

### DESCRIPTION OF THE DRAWINGS

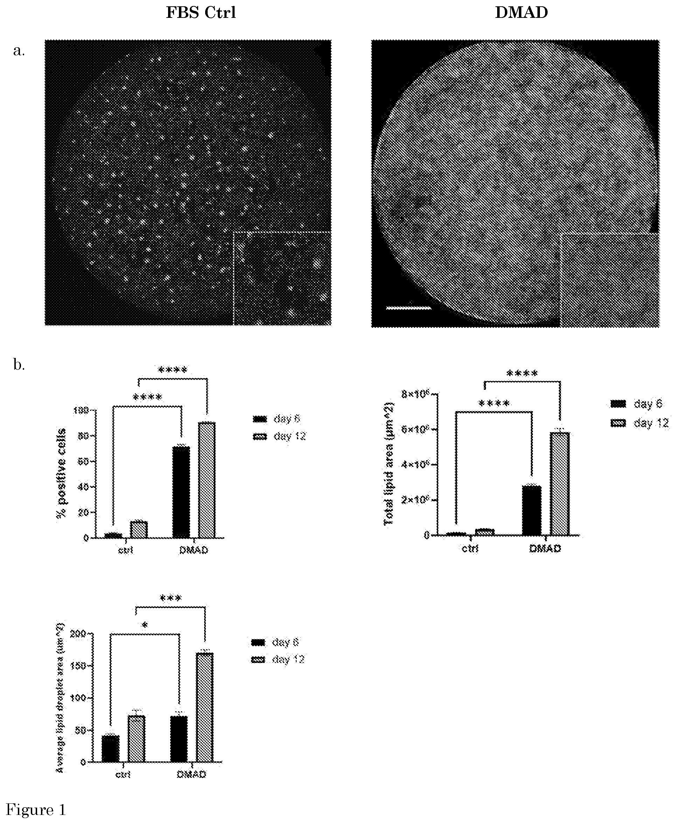

**Figure 1****: Mesenchymal stem cells (MSCs) differentiated in 2D with standard adipogenic media (ctrl) or with a serum-free adipogenic differentiation medium of the invention (DMAD).**
   (A) Representative HCA images of cells after 6 and 12 days of culture. Cells were stained with BODIPY and Hoechst, and images were taken with a ImageXpress Pico microscope. Scale bar: 1 mm.
   (B) Quantitative analysis of adipocyte cell differentiation using the HCA, showing % of positive cells, total lipid area and average area of lipid droplets. All numerical data is shown as mean ± sd; P-values: *, P ≤ 0.01; ***, P ≤ 0.001; ****, P ≤ 0.0001.

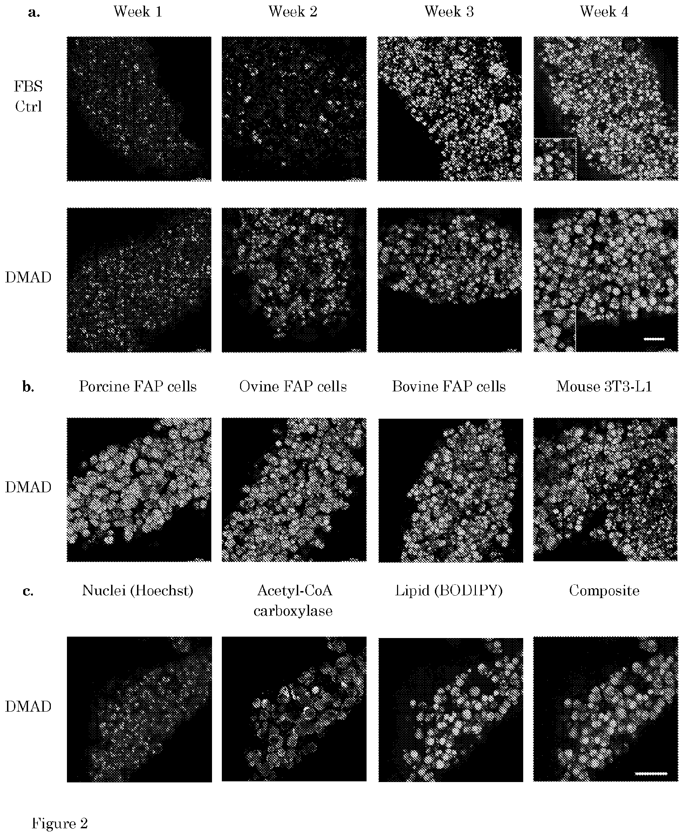

**Figure 2****: Immunohistochemical staining of 3D differentiating adipocytes.** Adipogenic precursors were encapsulated in alginate gel and differentiated for 4 weeks with a serum-free differentiation medium as disclosed herein (DMAD as disclosed in Example 1) or control medium, and representative confocal images (maximum projection) are shown to demonstrate the level of adipogenesis.
   (A) Bovine MSCs differentiated with control medium (top four panels) and DMAD (bottom four panels).
   (B) Porcine, ovine and bovine FAPs, and mouse 3T3-L1 after 4 weeks of culture with DMAD.
   (C) Bovine MSCs labeled with Hoechst, anti-ACC antibody and BODIPY, as well as a composite of all three channels. Scale bars: 100 µm.
**Figure 3****: Concentration testing of DMAD components in 2D.** FAP cells were cultured with 50% and 100% of the indicated DMAD component concentrations as disclosed in Example 1. Quantitative analysis of adipocyte cell differentiation using the HCA was carried out showing total lipid area of positive cells.
   vC: vitamin c, HC: hydrocortisone, Prog: progesterone. Data was normalised against the ctrl (100% of all compounds). All numerical data is shown as mean ± sd; P-values: *, P ≤ 0.05.
**Figure 4****: Adipogenic precursor cells differentiated in 2D in the absence of listed medium components.**
   Quantitative analysis of adipocyte cell differentiation using the HCA showing: (A) total lipid area of positive cells and (B) % of positive cells. Ctrl represents a medium of DMEM/F 12 with 3% FBS and supplemented with 4 conventional adipogenic inducers (IBMX (Sigma Aldrich-I5879; 0.5 mM), dexamethasone (Sigma Aldrich-D4902; 10 µM), rosiglitazone (Sigma Aldrich-R2408; 5 µM) and insulin (Peprotech-10-365; 10 µM)). All numerical data is shown as mean ± sd; P-values: **, P ≤ 0.01; ***, P ≤ 0.001; ****, P ≤ 0.0001.

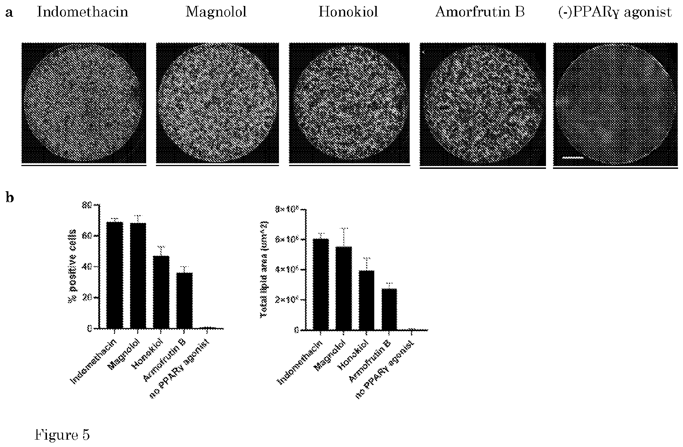

**Figure 5****: Adipogenic precursor cells differentiated in 2D with different PPAR_{Y} agonists.**
   (A) Representative HCA images of cells after 12 days of culture. Cells were stained with BODIPY and Hoechst, and images were taken with a ImageXpress Pico microscope. The graph on the right is a negative control showing differentiation in the absence of a PPAR_{Y} agonist.
   (B) Quantitative analysis of adipocyte cell differentiation using the HCA, showing % of positive cells and total area of lipids. Scale bar: 1 mm. All numerical data is shown as mean ± sd.

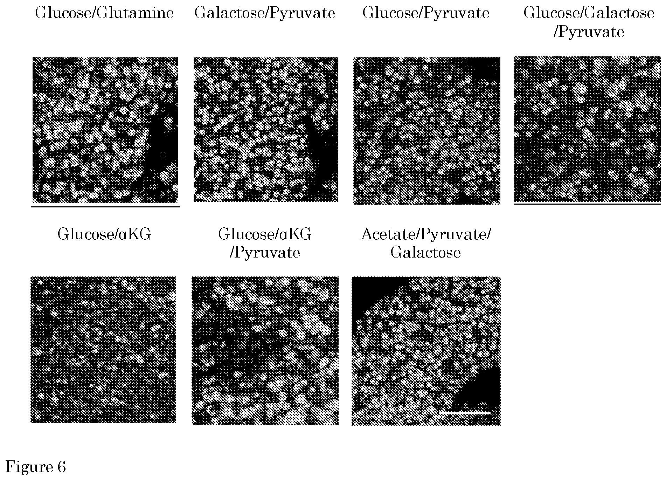

**Figure 6****: 3D differentiation of adipogenic precursor cells with various energy substrates.**
   Adipogenic precursor cells were encapsulated in alginate gel and differentiated for 4 weeks with DMAD supplemented with different energy building blocks, and representative confocal images (maximum projection) are shown to demonstrate levels of adipogenesis. Scale bar: 200 µm.

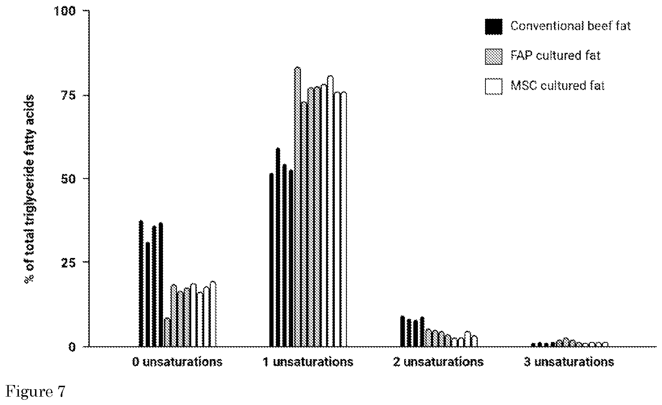

**Figure 7****: Lipidomics analysis of conventional cow fat and bovine cultured fat differentiated with a serum-free adipogenic differentiation medium as disclosed herein (DMAD as disclosed in Example 1).** Plot of relative percentage of acyl chains containing 0-3 saturations in the triglyceride lipid class. Four representative samples are shown per type of fat each from a different donor animal.

### DETAILED DESCRIPTION OF THE INVENTION

### Definitions

The terms 'differentiating' and 'differentiation', as used herein, includes reference to the process of specialization of cells. During this process, progenitor cells (which may also be referred to as precursor cells or stem cells) change from one cell type to another. Differentiation may be activated and regulated by hormones, growth factors or other signaling molecules. Preferably, the differentiation as referred to herein is adipogenic differentiation, i.e. the process wherein adipogenic progenitor cells ultimately differentiate into adipocytes (fat cells).

The term 'mammalian', as used herein, includes reference to any animal of the class Mammalia. Non-limiting examples of animals belonging to the class Mammalia are cattle, pigs, sheep, deer, and mice. A mammalian cell includes cells isolated from, derived from, differentiated from, expanded or originally found in an animal of the class Mammalia, and also includes cultured cells. A preferred mammal is a *Bos taurus.*

The term 'adipocyte', as used herein, can be used interchangeably with the term 'fat cell' and includes reference to a cultured fat cell or adipocyte or cultured fat tissue. Adipocytes may be categorized as forming white adipose tissue or brown adipose tissue. Adipocytes are found throughout the body. Adipocytes synthesize and store fat, including but not limited to lipids and triglycerides.

The term 'progenitor cell' as used herein, includes reference to a cell that is able to differentiate into a more specialized cell. The term 'progenitor cell' can be used interchangeably with the term 'precursor cell'. Progenitor cells may for example be stem cells, satellite cells, intermediate progenitor cells, radial glial cells, bone marrow stromal cells, periosteum, pancreatic progenitor cells, angioblasts, blast cells or adipogenic progenitor cells. No limitation to the stage of development is intended.

The term 'adipogenic progenitor cell', as used herein, includes reference to a cell that is able to differentiate into an adipocyte. Adipogenic progenitor cells may for example be pluripotent stem cells, induced pluripotent stem cells (iPSCs), mesenchymal stem cells, fibro-adipogenic progenitor (FAP) cells, lipoblasts, adipoblasts and preadipocytes. The adipogenic progenitor cell can for instance be derived from muscle or fat, and is in embodiments a muscle-derived adipogenic progenitor cell such as a (skeletal) muscle-derived fibro-adipogenic progenitor (FAP) cell. Preferably, the adipogenic progenitor cell is a mammalian adipogenic progenitor cell, for instance a bovine, ovine, porcine or murine, preferably bovine, adipogenic progenitor cell. FAP cells can be purified from muscle tissue samples by methods involving antigen-based cell sorting such as FACS. Cell surface markers expressed by bovine FAPs are CD9, CD14, CD49e (ITGA5), CD61 (ITGB3), CD140a (PDGFRA), and ITGA9. Bovine FAPs lack expression of hematopoietic marker CD45, endothelial marker CD321 (F11R), and the myogenic progenitor markers CD56 (NCAM1) and ITGA7. Preferably, the FAP cell is a bovine FAP cell.

The term 'culturing', as used herein, includes reference to the cell cultures of the cells disclosed herein and may refer, depending on the type of medium used, to propagation and/or proliferation (expansion) of adipogenic progenitor cells such as mammalian adipogenic progenitor cells or to differentiation of mammalian adipogenic progenitor cells into adipocytes. In the context of the invention, this term preferably includes reference to the differentiation of bovine adipogenic progenitor cells into bovine adipocytes. It should however be understood that a serum-free medium of the invention can also be employed to differentiate other non-human, mammalian adipogenic progenitor cells such as ovine, porcine or murine adipogenic progenitor cells. Therefore, any embodiment described herein in relation to bovine adipogenic progenitor cells, is also applicable to ovine (such as sheep), porcine (such as pig) or murine (such as mouse) adipogenic progenitor cells, i.e. progenitor cells of ovine, porcine or murine origin. Thus, in aspects and/or embodiments of a method for differentiation of the invention, a serum-free medium of the invention or a composition of the invention, instead of a bovine adipogenic progenitor cell, an ovine adipogenic progenitor, a porcine adipogenic progenitor or a murine adipogenic progenitor cell can be employed. In embodiments, the adipogenic progenitor cell is a muscle-derived or fat-derived adipogenic progenitor cell, such as a muscle-derived adipogenic progenitor cell e.g. a fibro-adipogenic progenitor (FAP) cell. The adipogenic progenitor cell can be derived from bovine skeletal muscle for instance by taking a biopsy. The term "culturing", as used herein in relation to a method for differentiating an adipogenic progenitor cell, includes reference to cell culture conditions that allow for adipogenic differentiation of a progenitor cell into a partially or terminally differentiated cell, preferably an adipocyte. The skilled person is well aware of suitable cell culture conditions that allow for differentiation of adipogenic progenitor cells, especially in light of the present disclosure.

The term 'animal-derived', as used herein, includes reference to components that are produced by an animal. Non-limiting examples of animal-derived components are fetal bovine serum, and components isolated from fetal bovine serum. Not animal-derived are for instance recombinantly produced animal proteins or peptides and any other component not produced by an animal but synthesized e.g. in the laboratory. If a medium as disclosed herein does not comprise any components or ingredients that are animal-derived, then the medium is animal component-free. Preferably, a serum-free medium as disclosed herein does not comprise components that are derived (obtained) from an animal.

The term "serum-free", as used herein, includes reference to a culture medium that is formulated in the absence of serum such as human serum or bovine serum or cell lysates of cells in said serum. A serum-free medium may contain serum proteins by way of supplementation of said serum protein such as serum albumin to said medium. However, preferably, all components of said medium are animal-free, i.e. that the components are not obtained from an animal but are for instance recombinantly produced. Preferably, a serum-free medium as disclosed herein is a chemically defined medium, i.e. a medium that is defined by the presence (and/or absence) of specific components. The concentrations of the components of a serum-free medium as disclosed herein can routinely be adjusted and optimized for the culturing and differentiation of adipogenic progenitor cells such as mammalian adipogenic progenitor cells, e.g. bovine, ovine or porcine adipogenic progenitor cells. The components in a serum-free medium of the invention are present in effective amounts that allow for differentiation of an adipogenic progenitor cell in culture, preferably wherein said adipogenic progenitor cell in a previous culturing step has been proliferated or expanded (or originates or is derived from a culture of progenitor cells that was previously proliferated or expanded) under serum-free conditions such as with a serum-free medium for proliferation of progenitor cells. A non-limiting example of a serum-free medium for proliferation or expansion of adipogenic progenitor cells is provided in Example 2 ("SF-PM" medium). A non-limiting example of a serum-free medium for differentiation of an adipogenic progenitor cell of the invention is provided in Example 1 ("DMAD" medium) and in other Examples.

The term 'hormone', as used herein, includes reference to members of a class of signal molecules from multicellular organisms that have an effect on physiology and/or behavior. Hormones bind to specific receptors in order to activate a signal transduction pathway. Examples of types of hormones are eicosanoids, steroids, amino acid derivatives, proteins, peptides and gases. Examples of hormones in the context of media for differentiation as disclosed herein are hydrocortisone and insulin.

The term 'cytokine', as used herein, includes reference to proteins that are involved in cell signaling, more specifically cell signaling related to or associated with the immune system and/or morphogenic pathways. Cytokines may be constituents of media, for example for the induction of expansion and/or differentiation. Examples of cytokines in the context of media for differentiation are molecules belonging to the group of bone morphogenic proteins (BMP).

The term 'growth factor', as used herein, includes reference to members of a class of signal molecules that generally have an effect on cell proliferation, growth and/or death. Growth factors may be constituents of media, for example for the induction of differentiation. Examples of cytokines in the context of media for differentiation are epidermal growth factor (EGF) and molecules belonging to the group of fibroblast growth factors (FGF).

The terms 'acceptable or suitable for animal consumption' and 'for animal consumption', as used herein, includes reference to components that are not harmful to healthy, non-allergic animals when consumed under normal circumstances and normal use. The term 'animal' may refer to any member of the kingdom Animalia, including humans and cats. Components (or ingredients) that are acceptable for human consumption can, amongst others, for instance be found in the Food Chemicals Codex, ISO 22000, or ingredients approved by the European Food Safety Authority or United States Food and Drug Administration. Compounds that are explicitly not suitable for animal consumption within the context of the invention, are rosiglitazone, isobutyl-methyl-xantane (IBMX) and dexamethasone.

The term "food-compatible", as used herein, includes reference to materials, ingredients or components that are non-toxic and safe for animal consumption, preferably for human consumption. The term "food-compatible" can be used interchangeably with the term "food-grade".

The terms 'peroxisome proliferator-activated receptor gamma', 'PPARG' and 'PPAR_{Y}', as used herein, include reference to a nuclear receptor that is present in cells of different tissue types such as adipose tissue, colon, rumen and placenta, and in macrophages. Alternative names of PPAR_{Y} include glitazone receptor and NR1C3.

The term 'PPAR_{Y} agonist', as used herein, includes reference to any chemical that binds to or interacts with PPAR_{Y}, and functions as an agonist. 'PPAR_{Y} agonist', as used herein, includes reference to endogenous agonists, full agonists, co-agonists, selective agonists, partial agonists, inverse agonists, superagonists, irreversible agonists and biased agonists. Non-limiting examples of the at least one PPARy agonist, which can be comprised in a serum-free medium for differentiating of the invention, include indomethacin, magnolol, amorfrutins (comprising, for example, amorfrutin 1, amorfrutin 2, amorfrutin A, amorfrutin B, amorfrutin C and amorfrutin D), honokiol, lecithine (such as L-α-lecithine from soy beans), formononetin, bixin, norbixin, catechin, Δ9-tetrahydrocannabinol, (9S, 13R)-12-oxo-phytodienoic acid, odoratin, hydroxy unsaturated fatty acids from *Coix lacrymajobi*, commipheric acid, kaempferol-3-O-β-glucopyranoside, citral, alkamides from *Echinacae purpera*, tocotrienols, deoxyelephantopin, acetylated flavonol glycosides, kampferol, quercetin, genistein, 5'-formulglabridin, (2R,3R)-3,4',7-trihydroxy-3'-prenylflavane, echinatin, (3R)-2',3',7-trihydroxy-4'-methoxyisoflavan, kanzonol X, kanzonol W, shinpterocarpin, licoflavanone A, glabrol, shinflavanone, gancaonin L, glabrone, licochalcone E, flavonoids from *Glycyrrhiza uralensis,* 3-arylcoumarins from *Glycyrrhiza uralensis,* meranzin, fatty acids from *Lycium chinense,* lunularin, fatty acids from *Melampyrum pratense,* cucurbitane-type triterpene glycosides, polyacetylenes from *Notopterygium incisum,* biochanin A, ginsenoside 20(S)-protopanaxatriol, ginsenoside Rb₁, fatty acids from *Pinellia ternata*, oleaninic acid, pseudolaric acid B, daidzein, amorphastilbol, carnosic acid, carnosol, 12-O-methul carnosic acid, α-linolenic acid, linoleic acid, naringenin, saurufuran A, isosilybin A, gallotannins, carvacrol, isoflavones from *Trifolium pratense,* ellagic acid, epicatechin gallate, flavonoids from *Vitis vinifera,* dehydrotrametenolic acid and 6-shogaol

The term "biogenic amine", as used herein, includes reference to a molecule containing one or more amine groups. Included in this group of biogenic amines are monoamines and polyamines. Within the group of monoamines are included ethanolamine, and within the group of polyamines are included agmatine, cadaverine, putrescine, spermine and spermidine. Preferably, the medium as disclosed herein comprises a at least biogenic amine that is a polyamine. Examples of the group of polyamines are putrescine, agmatine, cadaverine, spermine and spermidine. Preferably, the medium as disclosed herein comprises at least putrescine.

The term 'putrescine', as used herein, includes reference to a compound represented by the formula NH₂(CH₂)₄NH₂. Putrescine is otherwise referred to as tetramethylenediamine, butane-1,4-diamine or 1,4-diaminobutane. Putrescine may be produced chemically or biochemically.

The terms 'proliferating' and 'proliferation', as used herein, includes reference to expansion of adipogenic progenitor cells, i.e. to increase their cell number. During proliferation, cells are propagated.

The term 'source of', as used herein, includes reference to a medium component that can be provided as a precursor or derivative of said medium component, or is provided as the medium component as such. The skilled person is well aware of suitable precursors for medium components as described herein. For instance, L-alanyl-L-glutamine or glutamine can be used as a source of glutamine and glucose can be used as a source of glucose. Further, as an example, glucose can be provided as a source of energy.

The term 'bovine', as used herein, includes reference to any member of the subfamily Bovinae. The subfamily Bovinae includes the tribes Boselaphini, Bovini and Tragelaphini. Preferably, bovine as disclosed herein refers to the members of the subfamily Bovinae that are used for animal, preferably human, consumption. Non-limiting examples of such members include domestic cattle (*Bos taurus* and subspecies *Bos taurus taurus; Bos taurus indicus*)*,* banteng (*Bos javanicus*), gayal or mithun (*Bos frontalis*)*,* gaur (*Bos gaurus*)*, yak* (*Bos grunniens; Bos mutus*)*,* water buffalo (*Bubalus arnee*; *Bubalus bubalis*)*,* American bison (*Bison bison*)*,* kudu (*Tragelaphus strepsiceros; Tragelaphus imberbis*)*,* common eland (*Taurotragus oryx*)*,* giant eland (*Taurotragus derbianus*)*,* and nilgai (*Boselaphus tragocamelus*)*.* Especially preferred bovine species as disclosed herein are *Bos taurus* and its subspecies.

The term 'ovine', as used herein, includes reference to any member of the genus *Ovis.* Preferably, ovine as disclosed herein refers to the members of the genus Ovis that are used for animal, preferably human, consumption. A non-limiting example of such members includes domestic sheep (*Ovis aries*)*.*

The term 'porcine', as used herein, includes reference to any member of the genus *Sus.* Preferably, porcine as disclosed herein refers to the members of the genus Sus that are used for animal, preferably human, consumption. Non-limiting examples of such members include domestic pig (*Sus scrofa domesticus*) and wild boar (several *Sus scrofa* subspecies).

The term "murine", as used herein, includes reference to any member of the family *Muridae.* Preferably, murine as disclosed herein refers to the members of the family *Muridae* that are used for animal consumption. Non-limiting examples of such members include mice and rats.

The term 'suspension culture', as used herein, includes reference to different types of suspension culture such as microcarrier-based cell culture. Preferably, the expansion or proliferation of progenitor cells as described herein is performed using a microcarrier-based cell culture. A microcarrier-based cell culture preferably involves growing progenitor cells on the surface of microcarriers in suspension cultures. Alternatively, suspension culture refers to suspension cell culture, where cells are cultured in suspensions or aggregates and are not attached to a surface such as a microcarrier surface. In embodiments, expansion or proliferation of progenitor cells as described herein is performed by suspension cell culture.

The term 'hydrogel', as used herein, includes reference to crosslinked polymers with hydrophilic properties. Hydrogels may be formed by a variety of compounds, such as alginate, agarose, methylcellulose, hyaluronan, elastin-like polypeptides, collagen, chitosan, gelatin and starch. In embodiments, the hydrogel as disclosed herein comprises alginate.

The term 'fat product', as used herein, includes reference to cultured fat that is suitable for animal, preferably human, consumption. In embodiments, a cultured fat product can be distinguished from a natural animal-derived fat product, amongst others, by the absence of for instance immune cells such as antigen-presenting cells (APCs) such as monocytes or macrophages, T cells and B cells. Other distinguishing characteristics can be the absence of immune cell effector molecules such as antibodies, or red blood cells. Other distinguishing characteristics can be the absence of cartilage tissue, lower levels of fibrous tissue and/or absence of antibiotics and/or antibiotic residues.

The term 'meat product', as used herein, includes reference to cultured (artificial) meat that is suitable for animal, preferably human, consumption. A meat product generally comprises muscle tissue and preferably also fat tissue. In embodiments, a cultured meat product can, amongst others, be distinguished from a natural animal-derived meat product by the absence of for instance immune cells such as antigen-presenting cells (APCs) such as monocytes or macrophages, T cells and B cells. Other distinguishing characteristics can be the absence of immune cell effector molecules such as antibodies, or red blood cells. Other distinguishing characteristics can be the absence of cartilage tissue, lower levels of fibrous tissue and/or absence of antibiotics and/or antibiotic residues.

The phrase 'incorporating into a food product', as used herein, relates to the production of a food product using cultured fat cells optionally in combination with (cultured) muscle cells.

The term 'myocyte', as used herein, includes reference to a differentiated muscle cell, preferably a cultured muscle cell.

The term 'myotube', as used herein, includes reference to a multinuclear myocyte. Myotubes are generally formed by the fusion of myoblasts or myotube progenitor cells.

The terms 'myofiber' and 'muscle fiber', as used herein, include reference to a muscle cell in elongated or cylindrical form that comprises several myofibrils. A myofiber comprises one or more nuclei.

The term 'basal medium', as used herein, includes reference to a liquid medium that supports cellular growth by providing essential components for growth. A basal medium may be provided in liquid or powdered format. A basal medium that is not supplemented with any compound may enable cellular growth, but supplementation may be required for growth depending on the cell type. A basal medium may be supplemented with one or more components selected from the non-limiting group consisting of amino acids, lipids, sugars, carbohydrates, anions, cations, buffering agents, colorants, vitamins, antioxidants, hormones, enzymes, proteins and trace elements. Preferably, the basal medium as disclosed herein is a commercially available basal medium and may in itself already comprise a source of energy as disclosed herein.

The term 'DMEM', as used herein, includes reference to Dulbecco's Modified Eagle Medium, a basal medium. Without wishing to be exhaustive, DMEM may comprise amino acids, choline chloride, D-caldium pantothenate, folic acid, niacinamide, pyridoxine hydrochloride, riboflavin, thiamine hydrochloride, i-inositol, calcium chloride, ferric nitrate, magnesium sulfate, potassium chloride, sodium bicarbonate, sodium chloride, sodium phosphate monobasic, glucose and phenol red. Preferably, DMEM as disclosed herein is provided in combination with Ham's F-12 medium in a 1:1 mixture (referred to as DMEM/F-12).

The term 'Ham's F-12', as used herein, refers to Ham's F-12 nutrient mixture, a basal medium. Without wishing to be exhaustive, Ham's F-12 may comprise amino acids, D-biotin, choline chloride, folic acid, myo-inositol, niacinamide, d-pantothenic acid, pyrodoxin, riboflavin, thiamine, vitamin B₁₂, glucose, hypoxanthine, linoleic acid, phenol red, putrescine, pyruvic acid, thioctic acid, thymidine, sodium bicarbonate, calcium chloride, cupric sulfate, ferrous sulfate, magnesium chloride, potassium chloride, sodium bicarbonate, sodium chloride, sodium phosphate dibasic and zinc sulfate.

The terms 'epidermal growth factor' and 'EGF', as used herein, include reference to proteins that can stimulate cell growth and differentiation of animal cells, amongst other functions. The proteins belong to an epidermal growth factor family. EGFs bind to epidermal growth factor receptor (EGFR). EGFs are preferably recombinantly produced. Preferably, the EGF as disclosed herein is a recombinantly produced EGF, and can be a human EGF. It is to be understood that the terms 'epidermal growth factor' and 'EGF', as used herein, may also include reference to fragments of EGF that retain the biological function of EGF, such as EGF replacement peptides.

The terms 'fibroblast growth factor' and 'FGF', as used herein, include reference to proteins that can stimulate growth and differentiation of animal cells, amongst other functions. FGFs bind to a fibroblast growth factor receptor (FGFR). FGFs are preferably recombinantly produced. Preferably, the FGF as disclosed herein is a recombinant FGF, and can be a human FGF. Preferably, the FGF as disclosed herein is FGF2. It is to be understood that the terms 'fibroblast growth factor' and 'FGF, as used herein, may also include reference to fragments of FGF that retain the biological function of FGF, such as FGF replacement peptides.

The term 'insulin', as used herein, includes reference to peptide hormones that promote the uptake of glucose by cells, amongst other functions. Insulin is preferably recombinantly produced. Preferably, insulin as disclosed herein is a recombinant protein, and can be a human insulin.

The term 'hydrocortisone', as used herein, includes reference to a steroid hormone that is produced in animals. Hydrocortisone is involved in the fat metabolism of animals, amongst other functions. Hydrocortisone may otherwise be referred to as cortisol. Hydrocortisone may be obtained from animals, plants or microorganisms or may be chemically synthesized. In embodiments, hydrocortisone as disclosed herein is chemically synthesized or synthesized from plant sources. Preferably, hydrocortisone as disclosed herein is not derived from animals or animal material.

The term 'progesterone', as used herein, includes reference to a steroid hormone that is produced in animals. Progesterone may be obtained from animals, plants or microorganisms or may be chemically synthesized. In embodiments, progesterone as disclosed herein is chemically synthesized or synthesized from plant sources. Preferably, progesterone as disclosed herein is not derived from animals or animal material.

The term 'putrescine', as used herein, includes reference to a compound represented by the formula NH₂(CH₂)₄NH₂. Putrescine is otherwise referred to as tetramethylenediamine, butane-1,4-diamine and 1,4-diaminobutane. Putrescine may be produced chemically or biochemically.

The term 'ascorbic acid', as used herein, includes reference to a compound represented by the formula C₆H₈O₆. Ascorbic acid is otherwise referred to as vitamin C. Ascorbic acid may be produced chemically or biochemically. Also envisaged herein are derivates of ascorbic acid such as L-ascorbic acid 2-phosphate or salt forms thereof (e.g. L-Ascorbic acid 2-phosphate sesquimagnesium salt), which may be supplemented to a basal medium.

The terms 'bone morphogenetic protein' and 'BMP', as used herein, include reference to a group of growth factors that can be found in the animal body. This group comprises the BMP proteins BMP1, BMP2, BMP3, BMP4, BMP5, BMP6, BMP7, BMP8a, BMP8b, BMP10, BMP11 and BMP15. Preferably, the BMP as disclosed herein is BMP4. Preferably, the BMP as disclosed herein is recombinantly produced, and can be a human BMP. It is to be understood that the terms 'bone morphogenetic protein 4' and 'BMP4', as used herein, may also include reference to fragments of BMP4 that retain the biological function of BMP4, such as BMP4 replacement peptides.

The term 'lipid', as used herein in relation to a source of lipids, includes reference to hydrophobic or partially hydrophobic hydrocarbon molecules such as fatty acids, glycerolipids, glycerophospholipids, sphingolipids, sterols, prenols, saccharolipids and polyketides. Preferably, a source of lipids is provided as a (chemically defined) lipid concentrate, which can be a mixture of lipids in emulsion. Without wishing to be exhaustive, a chemically defined lipid concentrate as disclosed herein may comprise one or more of arachidonic acid, cholesterol, dl-alpha-tocopherol acetate, ethyl alcohol, linoleic acid, linolenic acid, myristic acid, oleic acid, palmitic acid, palmitoleic acid, stearic acid and/or Tween 80. In embodiments, a chemically defined lipid concentrate as disclosed herein comprises a combination of some or all the above-listed compounds.

The term 'thiazolidinedione', as used herein, can be used interchangeably with 'a compound of the thiazolidinedione family' and includes reference to a class of compounds comprising pioglitazone, rosiglitazone, lobeglitazone, ciglitazone, darglitazone, englitazone, netoglitazone, rivoglitazone, troglitazone and balaglitazone. Members of the thiazolidinedione family may also collectively be referred to as glitazones or TZDs. Preferably, compounds of the thiazolidinedione family are not comprised in a serum-free adipogenic differentiation medium as disclosed herein.

The term 'HEPES', as used herein, includes reference to 4-(2-hydroxyethyl)-1-piperazineethanesulfonic acid. HEPES is often used as a buffering agent. HEPES may be produced chemically or biochemically.

The term 'L-glutamate', as used herein, refers to the L form of 2-aminopentanedioic acid. L-glutamate is otherwise referred to as L-glutamic acid. L-glutamate may be provided as L-alanyl-L-glutamate. L-glutamate may be produced chemically or biochemically.

The term 'chemically defined lipid concentrate', as used herein, may include reference to a lipid emulsion. A chemically defined lipid concentrate as disclosed herein is an example of a lipid source as disclosed herein. Without wishing to be exhaustive, a chemically defined lipid concentrate as disclosed herein may comprise arachidonic acid, cholesterol, dl-alpha-tocopherol acetate, ethyl alcohol, linoleic acid, linolenic acid, myristic acid, oleic acid, palmitic acid, palmitoleic acid, stearic acid and/or Tween 80. In embodiments, a chemically defined lipid concentrate as disclosed herein comprises a combination of some or all the above-listed compounds.

The expression 'partially differentiated', as used herein, includes reference to cells that are in the process of differentiation.

The expression 'terminally differentiated', as used herein, includes reference to specialized cells that are under normal conditions no longer able to further differentiate and/or proliferate.

The term 'source of energy, as used herein, includes reference to a molecule that is involved as a substrate in a metabolism pathway of a mammalian cell that provides energy (such as energy in the form of ATP). The conversion of the energy source directly or indirectly leads to the generation of energy, for example in the form of ATP. In general, metabolites that are energy sources to a cell comprise carbon atoms. Examples of energy sources are sugars, proteins and fats.

The term 'energy metabolism pathway', as used herein, includes reference to any pathway of the mammalian cell that can be employed to generate energy from substrates involved in said pathway. Examples of energy metabolism pathways are glycolysis, glutaminolysis, mitochondrial respiration (also known as tricarboxylic acid cycle, citric acid cycle and Krebs cycle), pyruvate decarboxylation, ketosis and oxidative phosphorylation. An energy metabolism pathway leads, optionally in combination with other metabolism pathways, to the generation of ATP, which provides energy to multiple cellular processes.

The term 'triglyceride', as used herein, includes reference to a lipid that is derived from glycerol and three fatty acids. As a consequence, a triglyceride is an ester with three covalently linked fatty acids. These fatty acids may be saturated, characterized by one or more C=C double bond, or unsaturated, characterized by no C=C double bonds.

The term 'unsaturated fatty acid', as used herein, includes reference to fatty acids with at least one C=C double bond. Unsaturated fatty acids in animal fat and cultured fat may for example comprise 16, 18, 20 or 22 carbon atoms, and may for example comprise 1, 2, 3, 4, 5, or 6 C=C double bonds. Saturated fatty acids are fatty acids with no C=C double bond. Unsaturated and/or saturated fatty acids may be constituents of lipids, such as triglycerides. In general, unsaturated fatty acids are more beneficial to animal health than saturated fatty acids.

The term 'inflammatory cells', as used herein, includes reference to cells that are part of the immune system of animals. Examples include macrophages, neutrophils, dendritic cells, innate lymphoid cells, mast cells, eosinophils, basophils, natural killer cells, B cells, T cells and granulocytes.

The term 'antibiotics', as used herein, includes reference to antimicrobial substances that are active against bacteria, for example by killing bacteria or by inhibiting growth of bacteria. Antibiotics may be used in livestock, such as cattle. Antibiotics, or residues thereof, may be passed on to meat products after slaughtering of the animal. Examples of antibiotics used in cattle include bacitracin, bambermycin, laidlomycin, lasalocid, monensin, neomycin, and virginiamycin.

The term 'blood residues', as used herein, includes reference to components typically found in blood, such as serum, serum proteins, erythrocytes, leukocytes and thrombocytes.

The term 'microbial contamination', as used herein, includes reference to undesired presence of one or more microbe, such as bacteria, viruses, fungi and archaea. It also includes reference to pathogenic contamination of food products in general.

The term 'cartilage', as used herein, includes reference to elastic animal tissue that is for example found at the end of bones at joints, in the rib cage, in the ear and in the nose. The term 'cartilage' includes reference to the three types of cartilage, i.e. elastic cartilage, hyaline cartilage and fibrocartilage. Cartilage in meat products is not desirable for consumption.

The term 'fibrous tissue', as used herein, also referred to as fibrous connective tissue, includes reference to a tissue type with a high amount of fibers, such as elastic and collagenous fibers. In meat products, fibrous tissue may render the meat product tough and therefore less desirable for consumption.

### Methods for differentiating adipogenic progenitor cells

The invention provides a method for differentiating an adipogenic progenitor cell, comprising the step of: - culturing an adipogenic progenitor cell in a serum-free medium for differentiating an adipogenic progenitor cell as disclosed herein.

### Provision and isolation of an adipogenic progenitor cell

An adipogenic progenitor cell as disclosed herein is generally provided prior to differentiation. Providing an adipogenic progenitor cell as disclosed herein may be done in any form. The adipogenic progenitor cell as disclosed herein is in embodiments provided in the form of a tissue sample comprising said adipogenic progenitor cell. Said tissue sample may be taken from anywhere in the non-human animal body. In embodiments, said tissue sample is a muscle tissue or fat tissue sample, preferably a bovine muscle tissue sample.

For example, the tissue sample comprising adipogenic progenitor cells herein is obtained via biopsy on an animal.

The obtaining of the tissue sample, for example via biopsy, may be done using local anesthetic, for example at the biopsy site. Local anesthetic may for example be applied via subcutaneous injection, and may be done using procamidor.

For example, the biopsy as disclosed herein is taken via skin incision in order to expose muscle. From said muscle, a bovine muscle sample as disclosed herein can be taken of e.g. about one gram. The creation of the incision as disclosed herein and/or the obtaining of the bovine muscle sample as disclosed herein may for example be routinely made using a scalpel. Said bovine muscle sample can subsequently be collected on ice.

For example, the adipogenic progenitor cell as disclosed herein is obtained from a sample of semitendinosus muscleFor example, the adipogenic progenitor cell is obtained from a (skeletal) muscle sample that is derived from a cadaver, or from a living animal e.g. by taking a biopsy.

For example, the tissue sample as disclosed herein is subjected to enzymatic digestion such as with a collagenase to achieve dissociation of muscle fibers. For example, AFC A (Worthington, CLS-1, 2000 U/ml) may be used. Exemplary conditions for dissociation using collagenase are an incubation time of 45 minutes and an incubation temperature of 37°C.

Alternatively, or in combination with said step of enzymatic digestion, the tissue sample as disclosed herein may be incubated with erythrocyte lysis buffer. For example, 1x ACK erythrocyte lysis buffer may be used. Exemplary incubation conditions for erythrocyte lysis buffer are an incubation time of 1 minute and an incubation temperature of 37°C.

For example, after said step of isolating adipogenic progenitor cells from the tissue sample as disclosed herein, cells obtained from said tissue sample are resuspended and incubated in a serum-free proliferation medium such as the proliferation media described herein (e.g. medium SF-PM, see Example 2).

For example, cells obtained from a muscle tissue or fat tissue sample as disclosed herein after said enzymatic digestion treatment (e.g. with a collagenase) and/or said treatment that allows for erythrocyte lysis, can be precultured in a serum-free proliferation medium as disclosed herein and subsequently subjected to antigen-based cell sorting such as FACS in order to purify adipogenic progenitor cells.

For example, adipogenic progenitor cells that are MSCs can be provided in accordance with Example 1.

For example, adipogenic progenitor cells that are FAPs can be provided in accordance with Example 3.

### Purification of an adipogenic progenitor cell

An adipogenic progenitor cell, especially a FAP cell, as disclosed herein may be characterized and purified using antigen-based cell sorting, such as FACS, after isolation and optionally preculture. FAPs can be purified by antigen-based cell sorting, such as FACS, on the basis of (i) the presence of cell surface markers CD9, CD14, CD49e, CD61, CD140a and/or ITGA9, and/or (ii) the absence of cell surface markers CD45, CD321, CD56 and/or ITGA7. For example, at least 1, at least 2, at least 3, or at least 4 of said cell surface markers are selected from the lists of both (i) and (ii). For example, FAPs are purified by FACS on the basis of the presence of cell surface markers CD14, CD49e, CD61, CD140a and/or ITGA9, and/or (ii) the absence of cell surface markers CD56 and/or ITGA7. For example, FAPs are characterized by the presence of cell surface marker CD140a.

The term 'antigen-based cell sorting', as used herein, includes reference to any protocol that allows for sorting of cell types on the basis of the presence or absence of cell surface markers. Non-limiting examples of antigen-based cell sorting protocols are protocols that employ fluorescently labelled antibodies such as FACS, or protocols that are based on magnetic labelling or isotope labelling of the cell surface marker-binding antibodies. Preferably, the antigen-based cell sorting is fluorescence-activated cell sorting.

The term 'presence' or 'absence', as used herein in relation to antigen-based cell sorting such as FACS, includes reference to the discrimination between cell types on the basis of differential expression of cell surface marker proteins (antigens). The skilled person directly understands that the term 'absence' does not mean that protein expression of a cell surface marker protein is necessarily fully absent (i.e. zero), but that its expression is lower as compared to another cell type and is lower to such an extent that it allows for a negative selection criterion in cell sorting. Thus, antigen-based cell sorting such as FACS allows for discrimination between cell types on the basis of (i) cell surface markers that are expressed on a cell to such an extent that they can be used as a positive selection criterion (i.e. presence; "+") or (ii) cell surface markers that have a lower or reduced expression on a cell to such an extent that they can be used as a negative selection criterion (i.e. absence, "-").

### Expansion of an adipogenic progenitor cell

A method of the invention may further comprise the step of: - culturing an adipogenic progenitor cell in a serum-free medium for proliferating adipogenic progenitor cells, to thereby provide proliferated adipogenic progenitor cells. For example, proliferation takes place after isolation and purification of an adipogenic progenitor cell and prior to differentiation of an adipogenic progenitor cell.

Culturing resulting in the expansion of adipogenic progenitor cells may be performed two-dimensionally or three-dimensionally, i.e. as a two-dimensional or three-dimensional cell culture. For two-dimensional culturing, adipogenic progenitor cells as disclosed herein may be propagated in tissue culture flasks in a serum-free medium for proliferating adipogenic progenitor cells as disclosed herein. For three-dimensional culturing, adipogenic progenitor cells as disclosed herein may be propagated in any suitable cell culture vessel such as spinner flasks or bioreactors. Preferably, , culturing of adipogenic progenitor cells is performed in a way that leads to three-dimensional expansion. Preferably, microcarrier-based cell culturing is performed in order to expand progenitor cells.

An exemplary, but non-limiting, serum-free medium for proliferating adipogenic progenitor cells is described in Example 2 (medium "SF-PM"). In embodiments, a serum-free medium for proliferating an adipogenic progenitor cell as disclosed herein may comprise an albumin and a fibroblast growth factor (FGF, such as FGF2). For example, said serum-free medium for proliferating an adipocyte progenitor cell as disclosed herein may further comprises one or more vitamins and/or hormones selected from the group consisting of ascorbic acid or a derivative thereof, an insulin, a somatotropin and a hydrocortisone, and one or more cytokines and/or growth factors selected from the group consisting of a platelet-derived growth factor (PDGF), an insulin-like growth factor (IGF), a vascular endothelial growth factor (VEGF), an hepatocyte growth factor (HGF) and an interleukin 6 (IL-6). For example, said one or more cytokines and/or growth factors is selected from the group of combinations comprising i) an IL-6; ii) an IL-6 and an IGF; iii) an IL-6, an IGF and an HGF; iv) an IL-6, an IGF, an HGF and a PDGF; v) an IL-6, an IGF, and a VEGF; vi) an IL-6, an IGF and a PDGF; vii) an IL-6, a PDGF and a VEGF; viii) an IL-6, an IGF, a PDGF and a VEGF; and ix) an IL-6, an IGF, an HGF, a PDGF and a VEGF. For example, said serum-free medium for proliferating an adipogenic progenitor cells comprises a PDGF, a VEGF, an HGF, an IGF and an IL-6. Exemplary, but non-limiting, serum-free proliferation media, and expansion culture conditions, are for instance disclosed in PCT/NL2021/050066, the contents of which are incorporated herein by reference.

### Differentiating an adipocyte progenitor cell

Adipogenic differentiation of adipogenic progenitor cells as disclosed herein can occur two-dimensionally or three-dimensionally, i.e. as a two-dimensional or three-dimensional cell culture. Three-dimensional differentiation of adipogenic progenitor cells may result in a structure comprising adipocytes that from a macroscopic perspective mimics subcutaneous fat in texture and appearance. This is a desirable effect, as it may be used to create meat products that mimic non-cultured meat products in terms of texture and appearance.

For example, a cell culture vessel that allows for three-dimensional expansion is typically used. An example of a suitable culture vessel for three-dimensional expansion is a spinning flask (Corning), which may be coated, for example by siliconization. Siliconization may for example be done using Sigmacote (Sigma Aldrich), for example at a concentration of 10 cm²/mL.

Preferably, in order to achieve three-dimensional differentiation of adipogenic progenitor cells as disclosed herein, a three-dimensional system is provided that is preferably edible and scalable.

Preferably, adipogenic differentiation is performed as a three-dimensional cell culture, for instance in a protein matrix, scaffold or cell aggregate. For example, a hydrogel is used for three-dimensional cell culturing. For example, said hydrogel is a hydrogel comprising alginate.

For example, the step of culturing an adipogenic progenitor cell in a serum-free medium for differentiating an adipogenic progenitor cell is carried out in and/or on microfibres. These microfibres may for example be made using alginate (i.e. alginate-based microfibers). Thus, For example, the adipogenic progenitor cell is provided in the form of a three-dimensional cell culture (e.g. a microfiber, such as an alginate-based microfiber).

Exemplary conditions for three-dimensional differentiation of adipogenic progenitor cells as disclosed herein are as follows. Adipogenic progenitor cells are resuspended in 0.5% high viscosity alginate solution (Sigma, W201502) at a concentration of 3x10⁷ cells/mL. Cell-alginate suspension is injected into 66 mM CaCl2, 10 mM HEPES. Resultant microfibres are subsequently washed and transferred to a 12-well tissue culture plate containing a serum-free medium for differentiating an adipogenic progenitor cell as disclosed herein.

### Incorporating cultured adipocytes into a food product

For example for using the invention may comprise the step of: - incorporating cultured adipocytes into a food product for animal, preferably human, consumption.

For example, said food product is i) a cell-culture based fat product or ii) a cell-culture based meat product that comprises myocytes, myotubes and/or myofibers.

Said myocytes, myotubes and/or myofibers may be obtained by differentiating muscle progenitor cells. Preferably, said method for differentiating muscle progenitor cells into myocytes, myotubes and/or myofibers is entirely serum-free, more preferably entirely animal-free. The process of differentiating muscle progenitor cells into myocytes, myotubes and/or myofibers may be preceded by one or more of the steps of: - providing a muscle progenitor cell; - isolating a muscle progenitor cell; and - culturing/proliferating a muscle progenitor cell. The process of differentiating muscle progenitor cells into myocytes, myotubes and/or myofibers may be succeeded by the step of: - incorporating a differentiated muscle progenitor cell into a food product, preferably a food product that also comprises differentiated adipogenic progenitor cells.

### Serum-free medium for differentiating adipogenic progenitor cells of the invention

The invention also provides a serum-free medium for differentiating an adipogenic progenitor cell into an adipocyte for animal, preferably human, consumption. Preferably, the serum-free medium is used for differentiating a mammalian adipogenic progenitor cell, such as a bovine, ovine, porcine or murine adipogenic progenitor cell.

Preferably, a serum-free medium of the invention comprises at least one PPAR_{Y} agonist selected from the group formed by indomethacin, magnolol, amorfrutin B, honokiol, In embodiments, the at least one PPAR_{Y} agonist is indomethacin or magnolol. In embodiments, the at least one PPAR_{Y} agonist is indomethacin. Indomethacin, magnolol, amorfrutin B, and honokiol perform very beneficially as PPAR_{Y} agonists in a medium for differentiating an adipogenic progenitor cell as disclosed herein.

The at least one PPAR_{Y} agonist can be present in the medium in a concentration range of 0.05-5000 µM, more preferably 0.05-500 µM, most preferably 5-100 µM such as about 50 µM.

Preferably, a serum-free medium of the invention further comprises hormones such as hydrocortisone and insulin. Preferably, the hydrocortisone is an animal hydrocortisone, more preferably a mammalian hydrocortisone, such as human hydrocortisone (e.g. H0135 from Sigma Aldrich). The hormone that is a hydrocortisone can be present in the medium in a concentration of 0.01 - 1000 nM, preferably 1 - 500 nM, more preferably about 100 nM. The hydrocortisone as disclosed herein is preferably present in a serum-free medium as disclosed herein (or "of the invention", which terms can be used interchangeably herein) in combination with said at least one PPAR_{Y} agonist as disclosed herein.

Preferably, a serum-free medium of the invention further comprises insulin as one of the hormones. Preferably, the insulin as disclosed herein is animal insulin, more preferably mammalian insulin, such as recombinant human insulin (e.g. 10-365 from Peprotech). Insulin can be present in the medium in a concentration of 0.01-200 µM, preferably 0.1-20 µM, more preferably 1-2 µM. The insulin as disclosed herein is preferably present in a serum-free medium of the invention in combination with said at least one PPAR_{Y} agonist and said at least one hydrocortisone as disclosed herein.

Preferably, a serum-free medium of the invention further comprises at cytokines and/or growth factors selected from the group consisting of bone morphogenetic protein 4 (BMP4) and epidermal growth factor (EGF). Preferably, the BMP4 as disclosed herein is animal BMP4, more preferably mammalian BMP4, such as *E. coli* derived recombinant human BMP4 (e.g. 120-05ET from Peprotech). The BMP4 can be present in the medium in a concentration of 0.03-60000 nM, preferably 0.3-6000 nM, more preferably 3-600 nM or about 300 nM. The BMP4 as disclosed herein is preferably present in a serum-free medium of the invention in combination with said at least one PPAR_{Y} agonist and said hormones as disclosed herein. Preferably, the EGF as disclosed herein is animal EGF, more preferably mammalian EGF, such as recombinant human EGF (e.g. AF-100-15 from Peprotech). The EGF can be present in the medium in a concentration of 3-30000 pM, preferably 30-3000 pM, more preferably about 322 pM. In embodiments the EGF can be present in the medium in a concentration of at least 161 pM. The EGF as disclosed herein is preferably present in a serum-free medium of the invention in combination with said at least one PPAR_{Y} agonist and said hormones as disclosed herein.

In a preferred embodiment, a serum-free medium of the invention comprises BMP4 and EGF as disclosed herein.

In embodiments, a serum-free medium of the invention comprises BMP4, EGF and fibroblast growth factor (FGF). The FGF as disclosed herein is preferably an animal FGF, more preferably mammalian FGF, such as recombinant human FGF2 (e.g. 100-18B from Peprotech). The FGF as disclosed herein can be present in the medium in a concentration of 1-10000 pM, preferably 10-1000 pM, more preferably 20-800 pM, more preferably 50-500 pM, more preferably 75-250 pM, more preferably 100-150 pM, more preferably about 115 pM. The BMP4, EGF and fibroblast growth factor (FGF) as disclosed herein are preferably present in a serum-free medium of the invention in combination with said at least one PPAR_{Y} agonist and said hormones as disclosed herein.

Preferably, a serum-free medium of the invention further comprises an ascorbic acid or a derivative thereof such as L-ascorbic acid 2-phosphate. Ascorbic acid or a derivative thereof may be provided as a salt, such as L-ascorbic acid 2-phosphate sesquimagnesium salt hydrate (e.g. A8960-5G from Sigma Aldrich). Ascorbic acid or a derivative thereof such as L-ascorbic acid 2-phosphate can be present in the medium in a concentration of 0.01 - 10000 µM, preferably 1- 500 µM, more preferably about 227 µM. The ascorbic acid or a derivative thereof such as L-ascorbic acid 2-phosphate as disclosed herein is preferably present in a medium of the invention in combination with said at least one PPAR_{Y} agonist, said hormones, said FGF, said EGF and said BMP4 as disclosed herein.

In embodiments, a serum-free medium of the invention further comprises at least one biogenic amine such as a monoamine or a polyamine, e.g. a putrescine, ethanolamine, spermidine and/or spermine. Preferably, the biogenic amine is putrescine (e.g. 51799 from Sigma Aldrich). The biogenic amine can be present in the medium in a concentration of 0.01 - 1000 µM, more preferably 0.1 to 500 µM or 1-100 µM or 20-80 µM or 50-60 µM, most preferably about 56 µM. The biogenic amine as disclosed herein is preferably present in a serum-free medium of the invention in combination with said at least one PPAR_{Y} agonist, said hormones, said FGF, said EGF, said BMP4 and said ascorbic acid or a derivative thereof as disclosed herein.

In embodiments, a serum-free medium of the invention further comprises a source of lipids, for example a source of lipids comprising one or more of fatty acids, glycerolipids, glycerophospholipids, sphingolipids, sterols, prenols, saccharolipids and polyketides. Said source of lipids is preferably provided by a (chemically defined) lipid mixture such as a lipid concentrate or lipid concentrate CD (e.g. 11548846 from ThermoFisher Scientific). Preferably, the source of lipids, or chemically defined lipid concentrate, as disclosed herein comprises at least one selected from the group of linoleic acid, linolenic acid, myristic acid, oleic acid, palmitic acid, palmitoleic acid and stearic acid, or, in embodiments, a combination of linoleic acid, linolenic acid, myristic acid, oleic acid, palmitic acid, palmitoleic acid and stearic acid. For example, these lipids are present in a final concentration of approximately 10 µg/L each. For example, the source of lipids, e.g. chemically defined lipid concentrate, as used herein comprises arachidonic acid. For example, arachidonic acid is present in a final concentration of approximately 2 µg/L. For example, the chemically defined lipid concentrate as used herein comprises cholesterol. For example, cholesterol is present in a final concentration of approximately 220 µg/L. For example, the source of lipids, e.g. chemically defined lipid concentrate, as disclosed herein comprises DL-alpha-tocopherol acetate. in embodiments, DL-alpha-tocopherol acetate is present in a final concentration of approximately 70 µg/L. For example, the source of lipids, e.g. chemically defined lipid concentrate, as disclosed herein comprises tween 80^{®} (also referred to as polysorbate 80). For example, tween 80^{®} is present in a final concentration of approximately 2.2 µg/mL. The lipid source, e.g. lipid concentrate as disclosed herein is preferably present in a serum-free medium of the invention in combination with said at least one PPAR_{Y} agonist, said hormones, said growth factors and/or cytokines and said ascorbic acid or a derivative thereof as disclosed herein, and optionally said biogenic amine as disclosed herein.

In embodiments, a serum-free medium of the invention further comprises one or more basal media. Preferably, a serum-free medium of the invention further comprises one or more basal media selected from the group comprising DMEM and Ham's F-12; more preferably a combination of DMEM and Ham's F-12. For example, the combination of DMEM and Ham's F-12 is in a ratio of 1:10 to 10:1 v/v, more preferably in a ratio of about 1:1 v/v. The at least one basal medium as disclosed herein is preferably present in a medium of the invention in combination with said at least one PPAR_{Y} agonist, said at least one hydrocortisone, said hormones, said growth factors and/or cytokines and said ascorbic acid or a derivative thereof such as L-ascorbic acid 2-phosphate as disclosed herein, and optionally said biogenic amine and/or said lipid source as disclosed herein.

In embodiments, the serum-free medium of the invention may further comprise one or more additional vitamins and/or hormones, such as progesterone. Progesterone can be present in the medium in a concentration of 0.01 - 400 nM, preferably 0.1 - 40 nM, more preferably about 18 nM.

In embodiments, a serum-free medium of the invention may further comprise one or more buffering agent, such as one or more of sodium bicarbonate (e.g. S5761 from Sigma Aldrich) and/or HEPES (e.g. H3375 from Sigma Aldrich). The sodium bicarbonate can be present in the medium in a concentration of 0.2-2000 mM, preferably 2-200 mM, more preferably about 20 mM. The HEPES can be present in the medium in a concentration of 0.05-500 mM, preferably 0.5-50 mM, more preferably about 5 mM. The one or more buffering agent as disclosed herein is preferably present in a medium of the invention in combination with said at least one PPAR_{Y} agonist, said hormones, said growth factors and/or cytokines and said ascorbic acid or a derivative thereof such as L-ascorbic acid 2-phosphate as disclosed herein, and optionally said biogenic amine, said lipid source and/or said basal medium as disclosed herein.

A serum-free medium of the invention may further comprises one or more source of glutamine, preferably one or more of L-glutamine (e.g. G8540 from Sigma Aldrich) and L-alanyl-L-glutamine (e.g. GlutaMAX^{™} from Gibco). The one or more source of glutamine can be present in the medium in a concentration of 0.04-400 mM, preferably 0.4-40 mM, more preferably about 4 mM. The one or more source of glutamine as disclosed herein is preferably present in a medium of the invention in combination with said at least one PPAR_{Y} agonist, said hormones, said growth factors and/or cytokines and said ascorbic acid or a derivative thereof such as L-ascorbic acid 2-phosphate as disclosed herein, and optionally said biogenic amine, said lipid source, said basal medium and/or said buffering agent as disclosed herein.

A serum-free medium of the invention may further comprise one or more source of energy or carbohydrates such as glucose. The one or more source of carbohydrates can be present in the medium in a concentration of 0.02-2000 mM, such as 0.2-200 mM or 2-20 mM.

The skilled person can routinely calculate and adjust the osmolality of a solution in general and of a medium in particular if needed. Osmolality is typically expressed in milliosmoles per kilogram of water (mOsm/kg). Osmolality may be measured using an osmometer. In embodiments, the osmolality of a medium of the invention is within the range of 180-380 mOsm/kg, preferably within the range of 275-299 mOsm/kg.

In an embodiment, a serum-free medium of the invention comprises at least one source of energy that is selected from substrates involved in at least one energy metabolism pathway. Examples of energy metabolism pathways are glycolysis, glutaminolysis, mitochondrial respiration (also known as tricarboxylic acid cycle, citric acid cycle and Krebs cycle), pyruvate decarboxylation, ketosis, oxidative phosphorylation and pentose phosphate pathway. Examples of metabolites involved in energy metabolism pathways, which can function as energy sources, are monosaccharides, glucose, glucose 6-phosphate, fructose 6-phosphate, fructose 1,6-bisphosphate, dihydroxyacetone phosphate, glyceraldehyde 3-phosphate, 1,3-bisphosphoglycerate, 3-phosphoglycerate, 2-phosphoglycerate, phosphoenolpyruvate, pyruvate, glutamine, glutamate, aspartate, lactate, alanine, citrate, α-ketoglutarate, oxaloacetate, acetyl-CoA, citrate, cis-aconitate, isocitrate, oxalosuccinate, succinyl-CoA, succinate, fumarate, L-malate, galactose, fructose, xylose, sucrose, lactose, maltose, isomaltulose, trehalose and acetate.

In embodiments, said at least one energy source is an energy source selected from the group consisting of glucose, glutamine, galactose, pyruvate, acetate and alpha-ketoglutarate, or any combination thereof. In embodiments, said at least one energy source is a combination selected from the group of glucose and glutamine; glucose and galactose; glucose and pyruvate; glucose and acetate; glutamine and galactose; glutamine and pyruvate; glutamine and acetate; galactose and pyruvate; galactose and acetate; pyruvate and acetate; glucose, glutamine and galactose; glucose, glutamine and pyruvate; glucose, glutamine and acetate; glucose, galactose and pyruvate; glucose, galactose and acetate; glucose, pyruvate and acetate; glutamine, galactose and pyruvate; glutamine, galactose and acetate; glutamine, pyruvate and acetate; galactose, pyruvate and acetate; glucose, glutamine, galactose and pyruvate; glucose, glutamine, galactose and acetate; glucose, glutamine, pyruvate and acetate; glucose, galactose, pyruvate and acetate; glutamine, galactose, pyruvate and acetate; and glucose, glutamine, galactose, pyruvate and acetate. In embodiments, said at least one energy source is a combination of glucose and glutamine; a combination of galactose and pyruvate; a combination of glucose and pyruvate; a combination of acetate, pyruvate and galactose; a combination of glucose, galactose and pyruvate; a combination of glucose and alpha-ketoglutarate (aKG); and/or a combination of glucose, alpha-ketoglutarate (aKG) and pyruvate. In embodiments, said at least one energy source is a combination of galactose and pyruvate.

Said at least one energy source may substitute the energy source or energy sources that are present in a basal medium as disclosed herein.

For example, said at least one energy source is present in a concentration of 10 µM to 10 M, more preferably between 100 µM to 1 M, such as between 1 mM and 100 mM or between 2 mM and 21 mM. In embodiments, the total concentration of all energy sources selected from glucose, glutamine, galactose, pyruvate and acetate combined is between 2 and 200 mM, more preferably around 20 mM.

The invention also provides a composition comprising a serum-free medium for differentiating as disclosed herein and an adipogenic progenitor cell as disclosed herein and/or a partially or terminally differentiated cell obtained therefrom. Preferably, said adipogenic progenitor cell is a mammalian adipogenic progenitor cell, preferably a bovine, ovine, porcine or murine adipogenic progenitor cell.

### Fat products and meat products for animal, preferably human, consumption

The invention also provides a cultured fat product for animal, preferably human, consumption, comprising adipocytes obtainable by a method for differentiating an adipogenic progenitor cell of the invention.

A cultured fat product for animal, preferably human, consumption of the invention may be produced or processed to resemble known food products. Non-limiting examples of food products as disclosed herein are a hamburger, a sausage, a steak, minced meat, a meatball, corned beef, a charcuterie product, jerky or stewed meat. Food products also covers the combination of several types of food products. The adipocytes obtainable by a method of the invention as disclosed may be processed prior to or following incorporation into a food product. Non-limiting examples of processing as disclosed herein are boiling, grilling, freezing, pressing, salting, curing, fermenting, smoking, drying, canning, cutting, grinding, mixing, seasoning, tubing in casing and marinating. The cultured adipocytes as disclosed herein and the optional (cultured) mammalian myocytes, myotubes and/or myofibers as disclosed herein may be arranged in a specific manner in the food product, for example in order to create optical similarity with non-cultured food products and/or to improve texture.

For example, a cultured fat product for animal, preferably human, consumption of the invention contains between 0.01% and 70% cultured adipocytes as disclosed herein, more preferably between 1% and 30% cultured adipocytes as disclosed herein, more preferably between 5% and 20% cultured adipocytes as disclosed herein. Additionally to cultured adipocytes as disclosed herein, a cultured fat product as disclosed herein may comprise water, one or more salt, one or more fiber, one or more carbohydrate, one or more protein, one or more starch, one or more spice, one or more herb, one or more yeast extract, one or more casing ingredient, one or more vitamin, one or more oil, one or more hydrocolloid, one or more thickening agent, one or more preservative, one or more colorant, one or more antioxidant, one or more acidity regulator, one or more stabilizer, one or more emulsifier, one or more flavor enhancer and/or one or more sweetener. Preferably, all constituents of the fat product are animal-free. For the purpose of clarity and a concise description, features are described herein as part of the same or separate embodiments, however, it will be appreciated that the disclosure includes embodiments having combinations of all or some of the features described.

### EXAMPLES

### Example 1: Production of a serum-free medium of the invention

An exemplary chemically defined, serum-free medium for adipogenic differentiation (also referred to as "DMAD") of the invention was prepared as follows:
DMEM/F12 medium was supplemented with PSA (17-745E from Lonza) at 1%, HEPES (H3375 from Sigma Aldrich) at 4.9mM, hydrocortisone (H0135 from Sigma Aldrich) at 0.1 µM, insulin (recombinant human insulin, 10-365 from Peprotech) at 1-2 µM, lipid concentrate (chemically defined lipid concentrate, 11548846 from Thermo Fisher) at 0.001% (v/v), putrescine (51799 from Sigma Aldrich) at 56 µM, EGF (recombinant human EGF, AF-100-15 from Peprotech) at 322 pM, FGF2 (recombinant human FGF2, 100-18B from Peprotech) at 115 pM, progesterone (P8783 from Sigma Aldrich) at 17.8nM, ascorbic acid (L-Ascorbic acid 2-phosphate sesquimagnesium salt hydrate, A8960-5G from Sigma Aldrich) at 227 µM, indomethacin (I7378 from Sigma Aldrich) at 50 µM and BMP4 (Recombinant Human BMP-4 (E.coli derived), 120-05ET from Peprotech) at 300 nM.

Further, concentration ranges around the concentration values of the components mentioned above still providing for effective adipogenic differentiation were routinely established.

### Example 2: 2D adipogenic differentiation of adipogenic progenitor cells

### Materials and methods

### Isolation and proliferation of progenitor cells

Bovine mesenchymal stem cells (MSC) were isolated from bovine adipose tissue (*Bos taurus*) using a standard enzymatic method (Mehta et al., in Myogenesis (ed. Rønning, S. B.) vol. 1889 111-125 (Springer New York, 2019), and plated onto plastic tissue culture flasks to allow adherent cells to attach. MSCs were propagated in a serum-free proliferation medium ("SF-PM") for at least 10 population doublings prior to differentiation. Briefly, the cells were seeded at a density of 3000 cells/cm² in SF-PM in a collagen-coated cell culture flask and passaged every time they reach 80-90% confluence. The serum-free proliferation medium referred to as "SF-PM" contains: albumin (5 mg/ml), somatotropin (2 ng/ml), L-Ascorbic acid 2-phosphate (50 pg/ml), hydrocortisone (36 ng/ml), α-linolenic acid (1 pg/ml), insulin (10 pg/ml), transferrin (5.5 µg/ml), sodium selenite (0.0067 pg/ml), ethanolamine (2 µg/ml), L-alanyl-L-glutamine or glutamine (2mM), IL-6 (5 ng/ml), FGF2 also referred to as bFGF (10 ng/ml), IGF1 (100 ng/ml), VEGF (10 ng/ml), HGF (5 ng/ml), PDGF-BB (10 ng/ml) and DMEM / F12 basal medium.

### Adipogenic differentiation

At 90% confluence, proliferated MSCs were trypsinized and seeded in a 96-well collagen coated cell culture plate at 30,000 cells/cm² in SF-PM. After 24 hours, adipogenesis was induced by changing SF-PM to DMAD (the serum-free differentiation medium as depicted in Example 1), which was maintained on the cells for a period of 12 days with media changes every 3-4 days. As a control differentiation medium (ctrl), DMEM/F 12 with 3% FBS was supplemented with 4 conventional adipogenic inducers (IBMX (Sigma Aldrich-I5879; 0.5 mM), dexamethasone (Sigma Aldrich-D4902; 10 µM), rosiglitazone (Sigma Aldrich-R2408; 5 µM) and insulin (Peprotech-10-365; 10 µM)). All 4 inducers were used for the first 3 days of culture and only insulin and rosiglitazone were used for the remaining 9 days.

### Immunofluorescence analysis

Cells were fixed on days 6 and 12 with 4% PFA for 20 minutes at room temperature and washed twice with PBS. Cells were then stained with Hoechst (ThermoFisher 493/503, 1:2000) and BODIPY (Sigma Aldrich 345880, 1:1000) in PBS for 30 minutes. Adipogenic differentiation was quantified using the ImageXPress Pico High Content Analyser (HCA; Molecular devices). The parameters measured were total area of lipid, lipid droplet average area and percentage of positive cells. For the percentage of positive cells, the HCA software assigned BODIPY immunoreactivity to the nearest nuclei, (i.e. a positive cell) and the number of BODIPY positive nuclei was then divided by the total number of nuclei.

### Results

To assess if DMAD is able to induce adipogenic differentiation to a similar level as said conventional control differentiation medium (ctrl), MSCs were cultured in 2D for 12 days in DMAD and said 3% FBS control medium. Culturing cells in DMAD and said conventional control medium demonstrated that adipogenic differentiation could be achieved to a greater extent with DMAD than the control (Figure 1a). Quantitatively, a significantly higher percentage of cells were positive (6 times more) compared to the control (Figure 1b). The maturity of the adipocytes, which can be assessed by the size of lipid droplets, was doubled in cells differentiated in DMAD relative to control.

### Example 3: 3D adipogenic differentiation of adipogenic progenitor cells with a serum-free differentiation medium of the invention.

### Materials and methods

### Isolation and proliferation of adipogenic progenitor cells

### Cell isolation

Fresh bovine skeletal muscle samples were obtained from a registered abattoir according to national guidelines on animal tissue handling. Muscle-derived cells were isolated as previously described (Ding et al., Sci Rep. 8, 10808 (2018)). Briefly, bovine semitendinosus muscle was minced and dissociated with collagenase (CLSAFA, Worthington; 1 h, 37°C). Cell slurries were filtered through a 100 µm cell strainer and incubated in ammonium-chloride-potassium (ACK) erythrocyte lysis buffer (1 min, RT). Cells were resuspended in serum-free proliferation medium "SF-PM" as defined in Example 2, and filtered through a 40 µm strainer prior to culture.

A similar procedure was applied for porcine and ovine adipogenic progenitor cells.

### Fluorescence-activated cell sorting (FACS)

Prior to FACS, FAP cells were cultured for 72 h on bovine collagen type I (2.5 µg/cm²; Sigma-Aldrich, C2124) coated flasks and sorted using antigen-based sorting on the basis of the absence of expression of JAM1, CD45 and integrin alpha 7 (ITGA7), and the positive expression of integrin alpha 5 (ITGA5) or platelet derived growth factor alpha (PDGFRα; also known as CD140a). Unstained cells were used to define gating parameters, and sorting purities routinely checked by reanalysing the sorted fractions.

MSCs were obtained as described in Example 2.

### Three-dimensional adipogenic microfiber culture

Adipogenic progenitor cells (FAPs and MSCs) were resuspended in 0.5% high viscosity alginate solution (Sigma-Aldrich, W201502) at a concentration of 3x10⁷ cells/ml. The cell-alginate suspension was injected into 66 mM CaCl₂ in a 10 mM HEPES buffer. The formed microfibres were cultured in 12-well tissue culture plates for 28 days with the serum-free adipogenic differentiation media described in Example 1 (DMAD). As a control, DMEM/F12 with 3% FBS supplemented with 4 conventional adipogenic inducers (IBMX, dexamethasone, rosiglitazone, insulin) was used for 3 days, followed by DMEM/F 12 with 3% FBS supplemented with only rosiglitazone and insulin for 25 days. The media was replaced every 3-4 days for 28 days. All fibers were incubated on a shaking platform at 75 RPM at 37°C and 5% CO₂.

### Immunofluorescence for adipogenic microfibers

The differentiated microfibres were fixed using 4% FA in 66mM CaCl₂ buffered with 10mM HEPES for 1 h. Once fixed, the samples were washed in buffer solution I (66mM CaCl₂ buffered with 10mM HEPES) and stained overnight at 4°C (1: 125 BODIPY, 1: 1000 Hoechst). For immunocytochemistry, microfibres were then blocked/permeabilised in blocking solution (buffer solution I, 10% goat serum, 0.1% Triton X) for 1 h. Followed by incubation with acetyl-CoA carboxylase antibody (ACC; 1:200; Cell Signalling, #3676) overnight at 4°C and donkey anti-rabbit Alexa 594 secondary antibody (1:200, Thermo Fisher, A32754) for 2 h. Following 2 washes with buffer solution I, the microfibres were imaged on a confocal microscope (TCS SP8, Leica Microsystems) using a 25×/1.00 objective lens and 5 µm-Z steps.

### Results

In our 3D cell model, it is clear that bovine MSCs differentiated in DMAD demonstrate earlier onset of adipogenesis compared to the control medium, as well as a higher number of differentiated cells (Fig. 2a). In addition, lipid droplet size in cells differentiated with DMAD also appear larger than in the control medium after 4 weeks (Fig. 2a). In addition, it was seen that cells from various species (i.e. bovine, porcine, ovine and mouse) and cell types (i.e MSCs, fibro-adipogenic progenitor cells (FAPs) and fibroblast cell line), are able to differentiate into adipocytes with DMAD (Fig. 2b). These differentiated microfibres from FAP cells also stained strongly for acetyl-CoA carboxylase (ACC), in the cytoplasm, a marker of mature adipocytes (Fig. 2c).

### Example 4: Further exemplary serum-free adipogenic differentiation media of the invention

### Materials and methods

### Adipogenic differentiation

Adipogenic progenitor cells were cultured as described in Example 2, with the exception that single components (FGF, EGF, insulin, vitamin C, hydrocortisone, BMP4, lipid concentrate, HEPES, putrescine, progesterone) of the DMAD medium (the serum-free differentiation medium as depicted in Example 1) were removed. In addition, different concentrations (50% and 200%) were tested for EGF, FGF, vitamin C, hydrocortisone and progesterone.

### Results

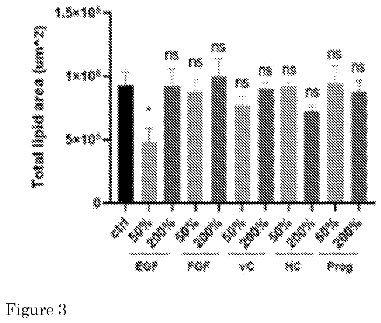

To assess if a range of concentrations of DMAD components can induce adipogenesis, 50% and 200% of the standard concentration (i.e. the concentration of indicated medium components as depicted in Example 1) was tested for EGF, FGF, vitamin C, hydrocortisone and progesterone. Decreasing the concentration of EGF resulted in significantly decreased adipogenesis (Fig. 3). For all other components, the range of concentrations tested demonstrated a similar effect on adipogenesis, as differences between 50% and 200% were negligible when compared to the 100% control. This led us to further investigate the necessity of DMAD components.

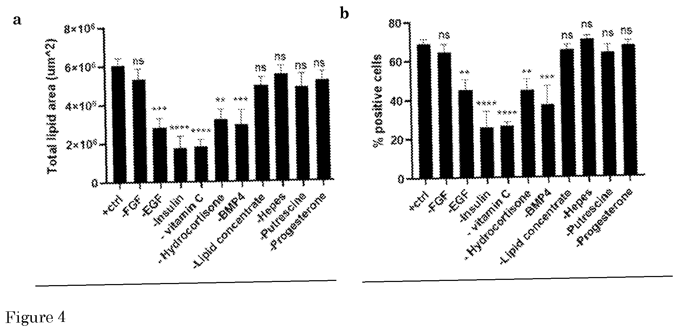

Removing FGF, lipid concentrate, HEPES, putrescine or progesterone from DMAD did not have significant effects on either the percentage of positive cells or the total lipid area. These components are thus not required for adipogenic differentiation (Fig. 4). On the other hand, the removal of insulin, vitamin C, hydrocortisone, BMP4 and EGF significantly decreased differentiation (Fig. 4). Besides a necessity for a PPAR_{Y} agonist (Fig. 5a), hormones such as insulin or hydrocortisone, ascorbic acid or a derivative thereof, and cytokines and/or growth factors BMP4 or EGF appear necessary in order to support adipogenic differentiation. It is further noted that antibiotics, such as PSA (a mixture of penicillin, streptomycin and amphotericin), can be omitted without affecting adipogenic differentiation potential.

### Example 5: PPAR_{Y} agonist testing

### Materials and methods

### Adipogenic differentiation

MSC cells were cultured as described in Example 2. With the exception that indomethacin (50 µM; I7378 from Sigma Aldrich) was replaced with one of the following three PPAR_{Y} agonists to test their efficiency: magnolol (M3445 from Sigma Aldrich) at 10 µM; honokiol (H4914 from Sigma Aldrich) at 10 µM and amorfrutin B (SMB00532 from Sigma Aldrich) at 10 µM.

### Results

Adipogenic precursor cells cultured in DMAD (the serum-free differentiation medium as depicted in Example 1) were able to undergo adipogenic differentiation with a range of PPAR_{Y} agonists, including indomethacin, magnolol, honokiol and amorfrutin B (Fig. 5). Magnolol and indomethacin led to the highest level of differentiation, followed by honokiol and amorfrutin B. In the absence of a PPAR_{Y} agonist, cells fail to differentiate, revealing the necessity of PPAR_{Y} agonists in DMAD.

### Example 6: Adipogenic differentiation using various energy substrates.

### Materials and methods

### Adipogenic differentiation

FAP cells were cultured as described in Example 3. With the exception that in the sugar/glutamine-free basal medium employed for the medium as depicted in Example 1, glucose (17 mM) and/or glutamine (2 mM) were replaced or combined with different combinations of monosaccharides such as galactose (17 mM), and substrates involved in energy metabolism such as glycolysis, glutaminolysis, and mitochondrial respiration including pyruvate (10 mM),acetate (10 mM), α-ketoglutarate (αKG; 5 mM).

### Results

To assess if different energy substrates can be used in DMAD to support differentiation, bovine FAPs were cultured with different combinations of glucose, glutamine, galactose, pyruvate, acetate and αKG. Our data demonstrates that DMAD can be successfully supplemented with various sugars and/or glutamine-sources to achieve robust adipogenic differentiation (Fig. 6).

### Example 7: Lipidomics profiling

### Materials and methods

### Mass spectrometry-based lipidomics

For the lipidomics sample preparation, 10-30mg of animal-derived fat tissue, as well as cultured fat tissue from both MSCs and FAPs as obtained in accordance with the previous examples were washed in PBS and stored at -80°C. Lipids were extracted using a modified Bligh-Dyer protocol (as described in Barniol-Xicota et al., Communications biology, 4:218 (2021)) and triglycerides were analysed by hydrophilic interaction liquid chromatography mass spectrometry (HILIC LC-MS/MS). To quantify the relative amount of each fatty acid within the triglyceride class, each fatty acid was normalised to the total amount of triglycerides present per sample and the total amount of protein.

### Results

Lipid profiles were analyzed in conventional fat tissue, and cultured fat from bovine FAP and MSC cells, from four donors each. Based on this lipidomics analysis, it was observed that a relatively similar fatty acid profile was found within the triglyceride class in all samples (Fig. 7). Though cultured fat revealed a higher relative percentage of unsaturated triglycerides compared to conventional fat tissue. Given the decisive role that triglyceride content plays in the taste of meat, these results suggest that a chemically defined serum-free media as disclosed herein is compatible with, and allows for, the production of cultured fat tissue for animal consumption.

## Claims

1. A method for differentiating a bovine, ovine, porcine or murine adipogenic progenitor cell, comprising the step of:
- culturing an adipogenic progenitor cell in a chemically defined serum-free medium for differentiating an adipogenic progenitor cell,
wherein the serum-free medium comprises:
- a basal medium;
- at least one peroxisome proliferator-activated receptor gamma (PPARy) agonist selected from the group consisting of indomethacin, amorfrutin B, magnolol and honokiol, preferably indomethacin or magnolol;
- two hormones consisting of insulin and hydrocortisone;
- two cytokines and/or growth factors consisting of bone morphogenetic protein 4 (BMP4) and epidermal growth factor (EGF); and
- ascorbic acid or a derivative thereof.

2. The method according to claim 1, wherein said serum-free medium comprises bone morphogenetic protein 4 (BMP4) and epidermal growth factor (EGF); and wherein said serum-free medium optionally further comprises fibroblast growth factor (FGF).

3. The method according to any one of the preceding claims, wherein said serum-free medium further comprises at least one biogenic amine such as putrescine.

4. The method according to any one of the preceding claims, wherein said serum-free medium further comprises a source of lipids such as a source of saturated and/or unsaturated fatty acids, preferably supplemented to said basal medium in the form of a concentrate.

5. The method according to any one of the preceding claims, wherein said basal medium is DMEM, Ham's F-12 or a mixture thereof.

6. The method according to any one of the preceding claims, wherein said serum-free medium does not comprise a differentiation inducer selected from the group consisting of isobutyl-methyl-xantane (IBMX), dexamethasone and/or a thiazolidinedione such as rosiglitazone, pioglitazone, lobeglitazone, cigilitazone, darglitazone, englitazone, netoglitazone, rivoglitazone, troglitazone and/or balaglitazone.

7. The method according to any one of the preceding claims, wherein said serum-free medium comprises a source of energy that allows for differentiation of said adipogenic progenitor cell wherein said source of energy is a substrate involved in at least one energy metabolism pathway such as glycolysis, mitochondrial respiration and/or pentose phosphate pathway and/or
wherein said source of energy is at least one sugar, preferably a monosaccharide or a disaccharide, such as glucose or galactose, optionally in combination with a source of glutamine, a pyruvate, acetate and/or alpha-ketoglutarate (aKG).

8. The method according to any one of the preceding claims, wherein the serum-free medium for differentiating comprises:
- indomethacin ;
- hydrocortisone;
- insulin;
- bone morphogenetic protein 4 (BMP4);
- epidermal growth factor (EGF);
- ascorbic acid or a derivative thereof; and
- a basal medium, preferably DMEM/F12;
and wherein said serum-free medium for differentiating optionally further comprises - putrescine, - a source of lipids, preferably wherein said source of lipids is a source of saturated and unsaturated fatty acids, - progesterone; and/or HEPES.

9. The method according to any one of the preceding claims, wherein the method for differentiating an adipogenic progenitor cell is a method for producing a cultured adipocyte for animal, preferably human, consumption by differentiating said adipogenic progenitor cell into an adipocyte.

10. The method according to any one of the preceding claims, wherein said method is a method for proliferating an adipogenic progenitor cell followed by differentiating proliferated adipogenic progenitor cells, wherein said method further comprises, prior to differentiating said adipogenic progenitor cell, a step of:
- culturing an adipogenic progenitor cell in a serum-free medium for proliferating adipogenic progenitor cells, to thereby provide proliferated adipogenic progenitor cells.

11. The method according to any one of the preceding claims, wherein said adipogenic progenitor cell is a mammalian adipogenic progenitor cell, preferably a bovine adipogenic progenitor cell such as a muscle-derived bovine adipogenic progenitor cell such as a fibro-adipogenic progenitor (FAP) cell.

12. The method according to any one of the preceding claims, wherein said culturing of said adipogenic progenitor cell in said serum-free medium for differentiating is performed under conditions that allow for differentiation of said adipogenic progenitor cell into an adipocyte.

13. The method according to any one of the preceding claims, further comprising the step of:
- incorporating said cultured adipocyte into a food product for animal, preferably human, consumption.

14. A serum-free medium for differentiating an adipogenic progenitor cell, wherein said medium is as defined in any one of the preceding claims.

## Patentansprüche

1. Verfahren zur Differenzierung von adipogenen Vorläuferzellen von Rindern, Schafen, Schweinen oder Mäusen, umfassend den Schritt:
- Kultivierung einer adipogenen Vorläuferzelle in einem chemisch definierten serumfreien Medium zur Differenzierung einer adipogenen Vorläuferzelle,
wobei das serumfreie Medium Folgendes umfasst:
- ein Basalmedium;
- mindestens einen Peroxisom-Proliferator-aktivierten Rezeptor-gamma (PPARγ)-Agonisten, ausgewählt aus der Gruppe bestehend aus Indomethacin, Amorfrutin B, Magnolol und Honokiol, vorzugsweise Indomethacin oder Magnolol;
- zwei Hormone, bestehend aus Insulin und Hydrocortison;
- zwei Zytokine und/oder Wachstumsfaktoren, bestehend aus Knochenmorphogenetischem Protein 4 (BMP4) und Epidermalem Wachstumsfaktor (EGF); und
- Ascorbinsäure oder ein Derivat davon.

2. Verfahren nach Anspruch 1, wobei das serumfreie Medium Knochenmorphogenetisches Protein 4 (BMP4) und Epidermalen Wachstumsfaktor (EGF) enthält und gegebenenfalls zusätzlich Fibroblasten-Wachstumsfaktor (FGF) enthält.

3. Verfahren nach einem der vorhergehenden Ansprüche, wobei das serumfreie Medium zusätzlich mindestens ein biogenes Amin wie Putrescin enthält.

4. Verfahren nach einem der vorhergehenden Ansprüche, wobei das serumfreie Medium zusätzlich eine Lipidquelle wie beispielsweise eine Quelle gesättigter und/oder ungesättigter Fettsäuren enthält, die dem Basismedium vorzugsweise in Form eines Konzentrats zugesetzt ist.

5. Verfahren nach einem der vorhergehenden Ansprüche, wobei das Basismedium DMEM, Ham's F-12 oder eine Mischung davon ist.

6. Verfahren nach einem der vorhergehenden Ansprüche, wobei das serumfreie Medium keinen Differenzierungsinduktor enthält, der aus der Gruppe bestehend aus Isobutylmethylxanthan (IBMX), Dexamethason und/oder einem Thiazolidindion wie Rosiglitazon, Pioglitazon, Lobeglitazon, Cigilitazon, Darglitazon, Englitazon, Netoglitazon, Rivoglitazon, Troglitazon und/oder Balaglitazon ausgewählt ist.

7. Verfahren nach einem der vorhergehenden Ansprüche, wobei das serumfreie Medium eine Energiequelle umfasst, die die Differenzierung der adipogenen Vorläuferzelle ermöglicht, wobei diese Energiequelle ein Substrat ist, das an mindestens einem Energiestoffwechselweg wie der Glykolyse, der mitochondrialen Atmung und/oder dem Pentosephosphatweg beteiligt ist, und/oder
wobei diese Energiequelle mindestens ein Zucker, vorzugsweise ein Monosaccharid oder ein Disaccharid wie Glucose oder Galactose, gegebenenfalls in Kombination mit einer Glutamin-, Pyruvat-, Acetat- und/oder α-Ketoglutarat-Quelle (αKG) ist.

8. Verfahren nach einem der vorhergehenden Ansprüche, wobei das serumfreie Medium zur Differenzierung Folgendes umfasst:
- Indomethacin;
- Hydrocortison;
- Insulin;
- Knochenmorphogenetisches Protein 4 (BMP4);
- Epidermaler Wachstumsfaktor (EGF);
- Ascorbinsäure oder ein Derivat davon; und
- ein Basalmedium, vorzugsweise DMEM/F12;
wobei das serumfreie Differenzierungsmedium gegebenenfalls zusätzlich Folgendes enthält:
- Putrescin,
- eine Lipidquelle, vorzugsweise eine Quelle gesättigter und ungesättigter Fettsäuren,
- Progesteron und/oder HEPES.

9. Verfahren nach einem der vorhergehenden Ansprüche, wobei das Verfahren zur Differenzierung einer adipogenen Vorläuferzelle ein Verfahren zur Herstellung eines kultivierten Adipozyten für den Verzehr durch Tiere, vorzugsweise Menschen, durch Differenzierung der adipogenen Vorläuferzelle zu einem Adipozyten ist.

10. Verfahren nach einem der vorhergehenden Ansprüche, wobei es sich um ein Verfahren zur Proliferation einer adipogenen Vorläuferzelle mit anschließender Differenzierung der proliferierten adipogenen Vorläuferzellen handelt, wobei das Verfahren vor der Differenzierung der adipogenen Vorläuferzelle weiterhin folgenden Schritt umfasst:
- Kultivierung einer adipogenen Vorläuferzelle in einem serumfreien Medium zur Proliferation adipogener Vorläuferzellen, um dadurch proliferierte adipogene Vorläuferzellen zu erhalten.

11. Verfahren nach einem der vorhergehenden Ansprüche, wobei es sich bei der adipogenen Vorläuferzelle um eine Säugetier-Vorläuferzelle handelt, vorzugsweise um eine Rinder-Vorläuferzelle, beispielsweise eine aus Muskelgewebe stammende Rinder-Vorläuferzelle wie eine fibroadipogene Vorläuferzelle (FAP-Zelle).

12. Verfahren nach einem der vorhergehenden Ansprüche, wobei die Kultivierung der adipogenen Vorläuferzelle in dem serumfreien Medium zur Differenzierung unter Bedingungen erfolgt, die die Differenzierung der adipogenen Vorläuferzelle zu einem Adipozyten ermöglichen.

13. Verfahren nach einem der vorhergehenden Ansprüche, ferner umfassend den Schritt:
- Einarbeiten des kultivierten Adipozyten in ein Lebensmittel für den Verzehr durch Tiere, vorzugsweise durch Menschen.

14. Serumfreies Medium zur Differenzierung einer adipogenen Vorläuferzelle, wobei das Medium wie in einem der vorhergehenden Ansprüche definiert ist.

## Revendications

1. Procédé de différenciation d'une cellule progénitrice adipogénique bovine, ovine, porcine ou murine, comprenant les étapes suivantes :
- culture d'une cellule progénitrice adipogénique dans un milieu sans sérum chimiquement défini, destiné à la différenciation de cette cellule,
ce milieu sans sérum comprenant :
- un milieu basal;
- au moins un agoniste du récepteur gamma activé par les proliférateurs de peroxysomes (PPARγ) choisi parmi l'indométacine, l'amorfrutine B, le magnolol et l'honokiol, de préférence l'indométacine ou le magnolol;
- deux hormones, l'insuline et l'hydrocortisone;
- deux cytokines et/ou facteurs de croissance, la protéine morphogénétique osseuse 4 (BMP4) et le facteur de croissance épidermique (EGF) ; et
- de l'acide ascorbique ou un de ses dérivés.

2. Procédé selon la revendication 1, dans lequel le milieu sans sérum comprend la protéine morphogénétique osseuse 4 (BMP4) et le facteur de croissance épidermique (EGF) ; et dans lequel ledit milieu sans sérum comprend optionnellement en outre le facteur de croissance des fibroblastes (FGF).

3. Procédé selon l'une quelconque des revendications précédentes, dans lequel ledit milieu sans sérum comprend en outre au moins une amine biogène telle que la putrescine.

4. Procédé selon l'une quelconque des revendications précédentes, dans lequel ledit milieu sans sérum comprend en outre une source de lipides telle qu'une source d'acides gras saturés et/ou insaturés, de préférence ajoutée au dit milieu de base sous forme de concentré.

5. Procédé selon l'une quelconque des revendications précédentes, dans lequel ledit milieu de base est du DMEM, du Ham's F-12 ou un mélange des deux.

6. Procédé selon l'une quelconque des revendications précédentes, dans lequel ledit milieu sans sérum ne comprend pas d'inducteur de différenciation choisi parmi l'isobutylméthylxantane (IBMX), la dexaméthasone et/ou une thiazolidinedione telle que la rosiglitazone, la pioglitazone, la lobeglitazone, la cigilitazone, la darglitazone, l'englitazone, la nétoglitazone, la rivoglitazone, la troglitazone et/ou la balaglitazone.

7. Procédé selon l'une quelconque des revendications précédentes, dans lequel ledit milieu sans sérum comprend une source d'énergie permettant la différenciation de ladite cellule progénitrice adipogénique, ladite source d'énergie étant un substrat impliqué dans au moins une voie métabolique énergétique telle que la glycolyse, la respiration mitochondriale et/ou la voie des pentoses phosphates, et/ou
dans lequel ladite source d'énergie est au moins un sucre, de préférence un monosaccharide ou un disaccharide, tel que le glucose ou le galactose, éventuellement en combinaison avec une source de glutamine, de pyruvate, d'acétate et/ou d'alpha-cétoglutarate (αKG).

8. Procédé selon l'une quelconque des revendications précédentes, dans lequel le milieu sans sérum de différenciation comprend :
- indométacine ;
- hydrocortisone ;
- insuline ;
- protéine morphogénétique osseuse 4 (BMP4) ;
- facteur de croissance épidermique (EGF) ;
- acide ascorbique ou un dérivé de celui-ci. et
- un milieu basal, de préférence DMEM/F12 ;
et dans lequel ledit milieu sans sérum pour la différenciation comprend optionnellement en outre :
- de la putrescine,
- une source de lipides, de préférence une source d'acides gras saturés et insaturés,
- de la progestérone;
et/ou du HEPES.

9. Procédé selon l'une quelconque des revendications précédentes, dans lequel le procédé de différenciation d'une cellule progénitrice adipogénique est un procédé de production d'un adipocyte cultivé destiné à la consommation animale, de préférence humaine, par différenciation de ladite cellule progénitrice adipogénique en un adipocyte.

10. Procédé selon l'une quelconque des revendications précédentes, **caractérisé en ce que** ce procédé consiste à multiplier une cellule progénitrice adipogénique puis à différencier les cellules progénitrices adipogéniques ainsi multipliées, ce procédé comprend en outre, avant la différenciation de ladite cellule progénitrice adipogénique, une étape consistant à :
- cultiver une cellule progénitrice adipogénique dans un milieu sans sérum destiné à la multiplication des cellules progénitrices adipogéniques, afin d'obtenir des cellules progénitrices adipogéniques multipliées.

11. Procédé selon l'une quelconque des revendications précédentes, **caractérisé en ce que** ladite cellule progénitrice adipogénique est une cellule progénitrice adipogénique de mammifère, de préférence une cellule progénitrice adipogénique bovine, telle qu'une cellule progénitrice adipogénique bovine d'origine musculaire, par exemple une cellule progénitrice fibro-adipogénique (FAP).

12. Procédé selon l'une quelconque des revendications précédentes, dans lequel la culture de la cellule progénitrice adipogénique dans le milieu sans sérum destiné à sa différenciation est réalisée dans des conditions permettant la différenciation de ladite cellule progénitrice adipogénique en adipocyte.

13. Procédé selon l'une quelconque des revendications précédentes, comprenant en outre l'étape suivante :
- l'incorporation de l'adipocyte cultivé dans un produit alimentaire destiné à la consommation animale, de préférence humaine.

14. Milieu sans sérum destiné à la différenciation d'une cellule progénitrice adipogénique, tel que défini dans l'une quelconque des revendications précédentes.
